# EUROPÄISCHE PATENTSCHRIFT

(11) **EP 2 300 079 B1**
(45) Veröffentlichungstag und Bekanntmachung des Hinweises auf die Patenterteilung: **13.05.2015**
(21) Anmeldenummer: 09765712.6
(22) Anmeldetag: 25.05.2009
(51) Int. Cl.: A61M 5/24, A61M 5/32, A61M 5/46

(54) **Ampulle mit ampullenhalterung**
Ampoule with ampoule holder
Ampoule avec support d' ampoule

(30) Priorität: 24.05.2008 DE 102008025011
(43) Veröffentlichungstag der Anmeldung: 30.03.2011
(73) Patentinhaber: Tecpharma Licensing AG, 3401 Burgdorf (CH)
(72) Erfinder: DRUNK, Anette, CH-3007 Bern (CH); BÜTTLER, Marius, CH-4702 Oensingen (CH); SCHIFFMANN, Frank, CH-3400 Burgdorf (CH); TRETOLA, Sara, CH-4562 Biberist (CH); WEBER, Kurt, CH-4556 Bolken (CH); MOSER, Ulrich, CH-3412 Heimiswil (CH); HIRSCHEL, Jürg, CH-5000 Aarau (CH)
(86) Internationale Anmeldenummer: PCT/EP2009/056333
(87) Internationale Veröffentlichungsnummer: WO 2009/153132

(56) Entgegenhaltungen:
- WO-A1-2004/004812
- WO-A2-2006/090188
- US-A1- 2008 051 729

## Beschreibung

Die Erfindung betrifft eine Ampullenhalterung zur Aufnahme einer Ampulle. Bei der Ampulle handelt es sich um ein Produktbehältnis aus der Medizintechnik, in der ein mit einer Injektionsvorrichtung zu verabreichendes Produkt enthalten ist. Bei der Ampulle kann es sich sowohl um eine Einkammerampulle als auch um eine Zweikammerampulle handeln.

Aus dem Stand der Technik sind Ampullen bekannt, die eine zylindrische Wandung aufweisen, die einen Kolben umgibt, der dichtend an der Innenseite der Wandung anliegt. Der Kolben ist relativ zur Wandung in Richtung eines Auslasses verschiebbar. Zwischen Kolben und Auslass befindet sich das Produkt. Am Auslass ist das Produktbehältnis mit einem Verschluss verschlossen. Der Verschluss kann ein sog. Septum sein, das mittels einer Nadel durchstechbar ist. Die bekannten Ampullen weisen zwischen der zylindrischen Gehäusewandung und dem Verschluss einen Ampullenhals auf, an dem sich die Ampulle verjüngt Die bekannten Verschlüsse haben einen kleineren Außendurchmesser als die zylindrische Gehäusewandung. Der kleinere Durchmesser des Verschlusses dient dazu, dass die Ampulle von der proximalen Öffnung einer Ampullenhalterung her in die Ampullenhalterung eingeführt werden kann und mit dem sich verjüngenden Teil des Ampullenhalses in einen festen Sitz mit der Ampullenhalterung gedrückt werden kann. Der kleine Durchmesser des Verschlusses ermöglicht das Durchstechen des Septums mit einer relativ dünnen Nadel. Dünne Nadeln haben zwar den Vorteil, dass sie beim Patienten weniger Einstechschmerzen erzeugen, aber den Nachteil, dass die zum Ausschütten auf den Kolben aufzubringende Ausschüttkraft relativ hoch ist, da das Produkt durch einen dünnen Kanal der Nadel gepresst werden muss. Dieser Effekt wird umso stärker, je höher die Viskosität des in der Ampulle enthaltenen Produkts ist.

Es ist eine Aufgabe der Erfindung, eine Ampullenhalterung anzugeben, mit der eine solche Ampulle gehalten werden kann.

US2008/0051729 beschreibt eine Ampullenhalterung umfassend eine Ampullenaufnahme und ein Befestigungsglied, wobei eines aus Befestigungsglied und Ampullenaufnahme ein Rastmittel und das andere ein Rastgegenmittel aufweist, wobei das Rastmittel und das Rastgegenmittel in einem Eingriff sind oder beim Befestigen des Befestigungsglieds in einen Eingriff bringbar sind.

Die Aufgaben werden gelöst durch die Merkmale des unabhängigen Anspruch 1. Vorteilhafte Weiterentwicklungen ergeben sich aus den abhängigen Ansprüchen und der Beschreibung einschließlich der Figuren.

Die Erfindung geht aus von einem Produktbehältnis für ein insbesondere flüssiges Medikament zur Verabreichung in einen Patienten. Bei dem flüssigen Medikament kann es sich um eine homogene oder heterogene Flüssigkeit handeln. Die Flüssigkeit kann eine Dispersion, insbesondere eine Suspension oder Emulsion sein. Eine Suspension bezeichnet Systeme aus einer Flüssigkeit und darin suspendierten praktisch unlöslichen Festkörperpartikeln, wie z.B. Pulverpartikel. Unter einer Emulsion versteht man ein feinverteiltes Gemisch zweier verschiedener normalerweise nicht mischbarer Flüssigkeiten. Das Produktbehältnis ist geeignet für die Aufbewahrung von Flüssigkeit sowohl niedriger als auch hoher Viskosität.

Das Produktbehältnis kann eine Ampulle sein, von der auch der Begriff Karpule umfasst ist. Stellvertretend für Produktbehältnisse wird im Folgenden eine Ampulle beschrieben.

Die Ampulle umfasst ein Gehäuse, das insbesondere rotationssymmetrisch ist. Bevorzugt ist das Gehäuse, d.h. wenigstens ein Teil davon, zylindrisch oder rohrförmig. Die Ampulle weist an ihrem distalen Ende einen Verschluss auf, der so ausgestaltet sein kann, dass er von einer Nadel oder einem ähnlichen länglichen insbesondere spitzen Gegenstand durchstechbar ist. Die Ampulle, insbesondere deren zylindrisches Gehäuse, ist am proximalen Ende offen, so dass ein Antriebsglied, wie z.B. eine Kolbenstange, auf einen von dem Gehäuse gelagerten Kolben wirken kann.

In der Ampulle können ein oder mehrere Kolben aufgenommen sein. Somit kann es sich bei der Ampulle um eine Einkammerampulle mit einem einzigen Kolben oder um eine Mehrkammerampulle mit zwei in Längsrichtung hintereinander angeordneten Kolben handeln.

In einer Einkammerampulle ist das zu verabreichende fluide Produkt zwischen dem Kolben und dem Verschluss angeordnet. Das Produkt kann durch Verschieben des Kolbens in Richtung Verschluss durch den Verschluss ausgeschüttet werden. Der dichtend an der Innenwand des Gehäuses anliegende Kolben ist entlang des zylindrischen Teils des Gehäuses verschiebbar.

Bei einer Zweikammerampulle wird das Medikament vor einer Injektion erst aus zwei Bestandteilen zusammengemischt bzw. abgemischt. Die Zweikammerampulle weist in der Regel eine erste Kammer für einen ersten Bestandteil und eine zweite Kammer für einen zweiten Bestandteil auf, wobei im Ausgangszustand die beiden Bestandteile z.B. durch eine Membran oder einen Kolben voneinander getrennt sind. Die getrennte Lagerung der Bestandteile kann die Lagerfähigkeit des Medikaments erhöhen, da das abgemischte Medikament nur von relativ kurzer Haltbarkeit ist. Beim Abmischen werden die Bestandteile zusammengeführt. Das Abmischen kann z.B. durch das Einsetzen der Zweikammerampulle in eine für solche Ampullen angepasste Vorrichtung geschehen. Die erste Kammer für den ersten Produktbestandteil ist zwischen dem Verschluss und dem ersten Kolben angeordnet. Der zweite Produktbestandteil ist zwischen dem ersten Kolben und dem zweiten Kolben angeordnet. Der zweite Kolben befindet sich proximal des ersten Kolbens. Beide Kolben liegen dichtend an dem inneren Umfang des Gehäuses an und sind relativ zum Gehäuse in Längsrichtung verschiebbar.

In der ersten Kammer kann sich ein z.B. flüssiger, vorzugsweise jedoch fester wie z.B. partikel- oder pulverförmiger Produktbestandteil befinden. Durch die trockene Lagerung des Produktbestandteils in der ersten Kammer kann die Haltbarkeit des Produktbestandteils erhöht werden. In der zweiten Kammer befindet sich vorzugsweise ein fluides Produkt.

Die Zweikammerampulle kann einen sog. Bypass aufweisen, der eine radial nach außen ragende Vertiefung im zylindrischen Teil des Gehäuses aufweist. Die Vertiefung ist länglich ausgebildet, d.h. länger als breiter und weist insbesondere eine Länge auf, die größer ist als die Länge des ersten Kolbens.

Vorzugsweise ist eine Verschiebebewegung des zweiten Kolbens auf den ersten Kolben übertragbar, insbesondere über den in der zweiten Kammer befindlichen Produktbestandteil. In einer verschobenen Position des ersten Kolbens, in der sich der Bypass sowohl distal als auch proximal über dem ersten Kolben erstreckt, kann der zweite Kolben relativ zum ersten Kolben bewegbar sein. Hierdurch ist das Volumen zwischen ersten und zweiten Kolben verkleinerbar, wodurch der in der zweiten Kammer enthaltene Produktbestandteil über den Bypass in die distale, d.h. erste Kammer fliessen kann oder gepresst wird. Die hierdurch zusammengeführten Produktbestandteile können vermischt werden. Zur Verabreichung des Produktgemischs kann die distale Stirnseite des zweiten Kolbens an die proximale Stirnseite des ersten Kolbens anstoßen. Erster und zweiter Kolben können dann gemeinsam zum Ausschütten des Produktgemischs in distale Richtung bewegt werden.

Der Verschluss der Ampulle ist an deren distalen Ende gebildet. Der Verschluss kann ein durchstechbares Mittel umfassen, das sich beim Durchstechen plastisch aufweitet entsprechend des eingestochenen Gegenstands, wie z.B. einer Nadel oder einem Fortsatz einer Nadeleinheit. Nach dem Entfernen des Körpers, der den Verschluss durchstochen hat, kann der Verschluss nach plastischer Verformung offen bleiben. Bevorzugt weist der Verschluss ein Material auf, das beim Durchstechen elastisch verformbar ist, so dass es sich um den Durchmesser des eindringenden Gegenstands anschmiegt und fluiddicht anliegt. Beim Herausziehen des durchstechenden Gegenstands aus dem Verschluss kann sich dieser aufgrund dessen Elastizität wieder fluiddicht verschließen. Somit kann das Eindringen von Schmutz in und das Austreten von Produkt aus dem Produktbehältnis vermieden werden. Beim erneuten Einstechen eines Körpers durch den Verschluss kann sich dieser aufgrund seiner Elastizität erneut aufweiten und sich wieder fluiddicht an den Umfang des Stechmittels anschmiegen. Somit kann die Ampulle auch für Produktabgaben in mehreren Dosen vorgesehen sein, bei denen jeweils eine neue Nadeleinheit verwendet wird. Zwischen den Injektionen kann eine gebrauchte Nadeleinheit gegen eine neue ausgetauscht werden.

Der elastische Teil des Verschlusses kann als sog. Septum bezeichnet werden, das z.B. aus einem Kunststoff, aus Kautschuk oder einem anderen geeigneten Material gebildet sein kann.

Die Ampulle weist an ihrem zylindrischen Gehäuse einen Außendurchmesser auf. Der Außendurchmesser des zylindrischen Teils der Ampulle kann in etwa so groß sein, wie ein Innendurchmesser einer Ampullenaufnahme, so dass die Ampullenaufnahme den zylindrischen Teil des Gehäuses lagern oder führen kann.

Erfindungsgemäß ragt der Verschluss radial über den Durchmesser, d.h. die äußere Umfangsfläche des zylindrischen Teils der Ampulle. Z.B. kann der sich bei einem rotationssymmetrischen Verschluss ergebende Durchmesser größer sein als der Durchmesser des zylindrischen Teils des Gehäuses. Bevorzugt ist der Außendurchmesser des Verschlusses, der größte Durchmesser der gesamten Ampulle.

Der Verschluss kann zumindest das Septum aufweisen, das bevorzugt einen größeren Durchmesser aufweist als der zylindrische Gehäuseabschnitt bzw. radial über den zylindrischen Gehäuseabschnitt ragt. Der Verschluss kann außerdem eine am distalen Ende des Gehäuses der Ampulle gebildete, radial über den äußeren Umfang des zylindrischen Gehäuseteils ragende Abragung aufweisen. Die Abragung kann ringförmig umlaufen, so dass sie einen größeren Außendurchmesser aufweisen kann als der zylindrische Gehäuseteil. Die ringförmig umlaufende Abragung kann z.B. als Wulst bezeichnet werden. Die ringförmige Abragung kann unmittelbar oder mittelbar, wie z.B. über einen Abschnitt reduzierten Außendurchmessers, der z.B. als Ampullenhals bezeichnet werden kann, mit dem zylindrischen Gehäuseabschnitt vorzugsweise einteilig verbunden sein. Das Gehäuse, das bevorzugt aus dem zylindrischen Gehäuseteil, der ringförmig umlaufenden Abragung und optional dem Ampullenhals gebildet wird, kann z.B. aus Glas oder einem geeigneten insbesondere durchsichtigen Kunststoff gebildet sein.

Der Ampullenhals kann sowohl einen gegenüber dem Außendurchmesser des Verschlusses und dem Außendurchmesser des zylindrischen Teils reduzierten Außendurchmesser und/oder einen gegenüber dem Innendurchmesser des zylindrischen Gehäuseteils reduzierten Innendurchmesser aufweisen. Durch den reduzierten Innendurchmesser kann erreicht werden, dass der Kolben nur bis zum Ende des zylindrischen Teils verschiebbar ist. Die Länge und Position des Ampullenhalters kann z.B. so angepasst sein, dass die Öffnung und/oder die Spitze eines durch den Verschluss eingeführten Stechmittels axial im Bereich des Ampullenhalses und/oder des Verschlusses zu liegen kommen. Somit kann vermieden werden, dass der Kolben an das Stechmittel anschlägt oder zu fest gegen das Stechmittel gedrückt wird, wenn es durch das Septum in das Produktbehältnis eingeführt ist.

Der Verschluss, insbesondere auch der Wulst und das Septum, kann im Bereich des Verschlusses in etwa eine zylindrische Umfangsfläche aufweisen. Der Verschluss kann z.B. ein Element aufweisen, welches das Septum und das Gehäuse, insbesondere die ringförmig umlaufende Abragung, verbindet bzw. in einer Verbindung hält. Dieses Teil kann z.B. das Septum und den Wulst über den äußeren Umfang umgeben. Ferner kann das Element das Septum distal und den Wulst proximal umgreifen, um eine axiale, fluiddichte Sicherung des Septums an den Wulst zu erreichen. Vorteilhaft kann der Bereich eines verringerten Durchmessers des Ampullenhalses sein, da das Element den Wulst weiter radial nach innen umgreifen kann als ohne den Bereich reduzierten Durchmessers. Das Element kann ein Umformelement sein, das bei der Herstellung um den Wulst und das auf den Wulst aufgesetzte Septum entsprechend umgeformt wird. Das Element kann an der distalen Stirnfläche des Verschlusses einen Durchbruch aufweisen, der insbesondere kreisförmig ist, und einen unmittelbaren Zugriff auf das Septum zulässt.

Das Septum kann einen scheibenförmigen Bereich aufweisen, der in etwa den Durchmesser des Wulsts aufweist. Ferner kann das Septum einen hohlzylindrischen Bereich aufweisen, der proximal von dem scheibenförmigen Bereich abragt. Der hohlzylindrische Durchmesser kann z.B. einen Außendurchmesser aufweisen, der in etwa dem Innendurchmesser des Gehäuses im axialen Bereich des Wulsts entspricht. Somit kann der zylindrische Bereich des Septums als Zentrierhilfe dienen. Der durch den hohlzylindrischen Teil des Septums gebildete Innenraum oder Aussparung kann dazu dienen, eine oder mehrere Öffnungen des Stechmittels einer aufgesetzten Nadeleinheit aufzunehmen.

Der Bypass der Ampulle kann z.B. vom äußeren Umfang des zylindrischen Teils der Ampulle abragen.

Die Ampulle kann vorteilhaft mit einer Ampullenhalterung an einer Injektionsvorrichtung angeordnet werden, wobei die Ampullenhalterung eine Ampullenaufnahme und ein Befestigungsglied aufweist, wobei die radiale Abragung des Verschlusses zwischen Ampullenhalterung und Befestigungsglied angeordnet, insbesondere eingefasst ist. Hierdurch wird es ermöglicht, die Ampullenhalterung axial zu fixieren, d.h. für eine Bewegung in distale und proximale Richtung. Ein gegebenenfalls auftretendes axiales Spiel des Produktbehältnisses kann, wie weiter unten beschrieben wird, verringert oder sogar beseitigt werden.

Die Ampullenaufnahme kann z.B. hülsenförmig sein mit einem Innendurchmesser, der in etwa dem Außendurchmesser des zylindrischen Teils der Ampulle entspricht, so dass die Ampulle seitlich von der Ampullenaufnahme geführt wird. Die Ampullenaufnahme kann z.B. ein Gewinde aufweisen, insbesondere ein Außengewinde, das in ein entsprechendes Gegengewinde einer Injektionsvorrichtung eingreifen kann, so dass die Ampullenaufnahme axial in das Gehäuse verschiebbar ist, insbesondere mit einer kombinierten Drehbewegung. Statt des Gewindes können auch eine Axialführung oder andere Komponenten vorgesehen sein, solange sie die axiale Bewegung der Ampullenaufnahme in die Injektionsvorrichtung erlauben.

Das Befestigungsglied kann an der Ampullenaufnahme befestigt oder befestigbar sein. Z.B. kann das Befestigungsglied einteilig mit der Ampullenaufnahme gebildet sein. Bevorzugt sind die Ampullenaufnahme und das Befestigungsglied separate Teile, die zusammen die Ampullenhalterung bilden.

Das Befestigungsglied kann eine Abragung sein oder aufweisen, welche an eine Stirnseite des Verschlusses der Ampulle, insbesondere an die distale Stirnseite, angreift. Z.B. kann die Abragung ein Schnapper oder ein Nocken oder dergleichen sein, wobei sich die Abragung z.B. von aussen radial nach innen erstrecken kann.

Die Ampulle kann mit der proximalen Seite des Verschlusses an der Ampullenaufnahme anliegen, wie z.B. an der Stirnseite der Ampullenaufnahme oder an einer an der Ampullenaufnahme von einem inneren Umfang radial nach innen gerichteten Abragung. Bevorzugt weist die Ampullenaufnahme im Bereich, dort wo die Stirnseite des Verschlusses zur axialen Anlage kommen soll, einen Bereich mit einem verringerten Innendurchmesser auf, der kleiner ist als der Außendurchmesser des Verschlusses und bevorzugt größer ist als der zylindrische Teil des Gehäuses. Insbesondere kann eine Stirnfläche der Ampullenaufnahme, wie z.B. das distale Ende, den Anschlag für den Verschluss, insbesondere dessen proximales Ende bieten.

Es ist bevorzugt, dass die Ampulle mit ihrem proximalen Ende zuerst, d.h. mit ihrem zylindrischen Gehäuseteil voran, z.B. durch die proximale Stirnseite der Ampullenaufnahme in die Ampullenaufnahme einführbar oder eingeführt ist. Das Befestigungsglied kann z.B. beim Einführen der Ampulle ausgelenkt werden und in der vollständig eingeführten Position zurückschnappen, um die Ampulle axial in zumindest eine Richtung zu fixieren.

In bevorzugten Ausführungen kann das Befestigungsglied formschlüssig mit der Ampullenaufnahme verbunden werden, insbesondere dreh- und axialfest relativ zur Ampullenaufnahme. Beispielsweise können die Ampullenaufnahme und das Befestigungsglied ineinandergreifende Elemente aufweisen, die z.B. wenn das Befestigungsglied vollständig auf die Ampullenaufnahme aufgesetzt ist, ineinander greifen oder einrasten.

Für den Fall, dass statt einer Einkammerampulle eine Zweikammerampulle in die Ampullenaufnahme eingeführt wird, kann diese eine sich Richtung der Stirnseite her entlang der Ampullenaufnahme erstreckende Aussparung für den Bypass der Zweikammerampulle aufweisen. Beispielsweise können mehrere solche über dem Umfang verteilte Aussparungen vorgesehen sein, so dass die Ampulle in nahezu jeder Winkelposition in die Ampullenaufnahme einführbar ist. Die Aussparung für den Bypass kann in Umfangsrichtung wirkende Drehanschläge aufweisen, die eine Drehung der Ampulle relativ zur Ampullenaufnahme verhindern, wenn die Ampulle eingeführt ist.

In vorteilhaften Weiterbildungen kann zumindest eines aus Ampullenaufnahme und Befestigungsglied wenigstens eine gegen den Verschluss gerichtete Abragung, wie z.B. einen Nocken, aufweisen. Der wenigstens eine Nocken kann sich in Längsrichtung der Ampulle oder der Ampullenhalterung erstrecken, insbesondere in distale oder proximale Richtung. Bevorzugt liegt der Verschluss, insbesondere mit seiner Stirnseite, an den mindestens einen Nocken an. Durch die durch den Nocken hervorgerufene punktuelle Auflage für den Verschluss kann ein Spiel in der axialen Einfassung des Verschlusses noch besser verringert werden. Die mindestens eine Abragung kann starr oder federnd, insbesondere in Axialrichtung federnd, an der Ampullenaufnahme und/oder dem Befestigungsglied angeordnet sein. Die Abragung kann allgemein als Eingriffsglied bezeichnet werden.

Die mindestens eine Abragung, insbesondere Nocken, kann an der Ampullenaufnahme, nämlich dort, wo das vollständig eingeführte Produktbehältnis in axiale Anlage kommt, angeordnet sein, insbesondere an der Stirnfläche der Ampullenaufnahme. Alternativ oder zusätzlich kann die wenigstens eine Abragung an einer radial nach innen gerichteten Abragung des Befestigungsglieds gebildet sein. Insbesondere dort, wo das Befestigungsglied die Stirnseite des Verschlusses umgreift.

Die Ampullenhalterung, insbesondere die Ampullenaufnahme und/oder das Befestigungsglied, kann Mittel aufweisen, die die Befestigung einer Nadeleinheit an der Ampullenhalterung ermöglichen. Beispielsweise kann die Nadeleinheit aufgesteckt, wie z.B. mit einer konischen Fläche, oder aufgeschraubt, wie z.B. mit einem Gewinde, werden. Bei einer an der Ampulle angeordneten Nadeleinheit kann diese z.B. mit einem Befestigungsabschnitt an der Ampullenhalterung befestigt sein. Bei einer vollständig befestigten Nadeleinheit kann ein Stechmittel, wie z.B. ein Fortsatz oder eine Nadel, das Septum durchstechen und eine Fluidverbindung zwischen einer distalen Nadelspitze der Nadeleinheit und dem Produktbehältnis herstellen. Bevorzugt wird die Nadeleinheit an dem Befestigungsglied festgeschraubt, das z.B. ein Innengewinde aufweist, in das ein Außengewinde der Nadeleinheit eingreifen kann.

Die Ampullenaufnahme kann Mittel, insbesondere ein Gewinde aufweisen, mit den sie an einer Injektionsvorrichtung befestigbar und in befestigtem Zustand mit einer Axialbewegung, insbesondere kombiniert mit einer Drehbewegung, in die Injektionsvorrichtung bewegbar ist. Beispielsweise kann die Ampullenaufnahme ein oder mehrere Rastelemente aufweisen, die bei der Bewegung des Ampullenhalters in die Injektionsvorrichtung an bestimmten Positionen ein akustisches und/oder taktiles Signal erzeugen, indem z.B. das Rastelement über ein Gegenelement rastet.

Vorteilhaft kann das aufgrund des vom Durchmesser her vergrößerten Septums ein dickerer Fortsatz oder eine dickere Nadel zum Einstechen in das Septum verwendet werden, was bei herkömmlichen engen Septen nur eingeschränkt, nämlich nur mit einer dünnen Nadel möglich ist. Durch die dünne Nadel wird sich jedoch ein erhöhter Ausschüttwiderstand ergeben. Bevorzugt ist daher, dass die Nadeleinheit einen Nadelträger aufweist, von der ein Fortsatz in proximale Richtung abragt zum Durchstechen des Septums und eine Injektionsnadel in distale Richtung abragt, zum Einstechen in einen Patienten, wobei der Fortsatz einen größeren Durchmesser aufweist als die Injektionsnadel. Im Fortsatz und in der Injektionsnadel kann ein Fluidkanal enthalten sein, der das Produkt aus dem Produktbehältnis zur Injektionsnadelspitze transportiert. Der Fluidkanal kann im Bereich des Fortsatzes einen größeren Fluidführungsquerschnitt aufweisen als im Bereich der Injektionsnadel.

Bei der Ampullenhalterung, bei der der Verschluss bzw. dessen radiale Abragung zwischen Ampullenaufnahme und Befestigungsglied angeordnet oder anordenbar ist, ist es von besonderem Vorteil, wenn das Befestigungsglied das Eingriffsglied aufweist, das an das Produktbehältnis angreift oder in den Eingriff mit dem Produktbehältnis bringbar ist. Hierdurch kann erreicht werden, dass das Eingriffsglied mit einem definierten Anteil an das Produktbehältnis drückt. Insbesondere kann das Eingriffsglied federnd angeordnet sein. Dadurch kann das Eingriffsglied bei der Befestigung des Befestigungsglieds an der Ampullenaufnahme mit einer Federkraft gegen das Produktbehältnis drücken und somit das axiale Spiel des Produktbehältnisses zwischen Ampullenaufnahme und Befestigungsglied verringern oder sogar aufheben. Alternativ oder zusätzlich kann das federnde Eingriffsglied von einem Teil, insbesondere der an das Befestigungsglieds anbringbaren Nadeleinheit gegen den Verschluss des Produktbehältnisses gedrückt werden. Dadurch kann das Spiel noch besser verringert werden.

Für die federnde Anordnung des Eingriffsglieds hat sich als besonders vorteilhaft ein Arm erwiesen, der sich in Längsrichtung oder in Umfangsrichtung erstreckt, wobei das Eingriffsglied an einem Ende des Arms angeordnet und der Arm mit dem anderen Ende insbesondere einstückig an dem Befestigungsglied gebildet ist.

Bevorzugt kann das Befestigungsglied, insbesondere das Eingriffsglied, ein Rastelement aufweisen, das ein Lösen der Nadeleinheit von dem Befestigungsglied zumindest erschwert. Durch das Rastelement kann zum Lösen der Nadeleinheit ein höheres Drehmoment erforderlich sein als ohne Rastelement. Dadurch kann auf einfache Weise verhindert werden, dass die an dem Befestigungsglied angeordnete Nadeleinheit, versehentlich gelöst wird.

In der Ampullenhalterung ist das Befestigungsglied mit einer Axialbewegung ohne Drehbewegung auf die Ampullenaufnahme verrastbar.

Insbesondere können eines aus Befestigungsglied und Ampullenaufnahme ein Rastmittel und das andere aus Befestigungsglied und Ampullenaufnahme ein Rastgegenmittel aufweisen, wobei das Rastmittel und das Rastgegenmittel in einem Eingriff sind oder beim Befestigen des Befestigungsglieds bringbar sind. Hierdurch wird das Befestigungsmittel insbesondere unlösbar mit der Ampullenaufnahme verbunden. Zur Verringerung des axialen Spiels des Verschlusses zwischen Befestigungsglied und Ampullenaufnahme können Rastmittel und Rastgegenmittel so gestaltet sein, dass, wenn sie ineinander greifen, das Befestigungsglied in diskreten Schritten in Befestigungsrichtung bewegt werden kann und in Gegenrichtung nicht bewegbar ist. Das Befestigungsglied, insbesondere das Eingriffsglied, kann in diskreten Rastschritten so weit in Richtung Verschluss bewegt werden, bis das Befestigungsglied oder das Eingriffsglied gegen den Verschluss drückt, so dass das axiale Spiel des Verschlusses aufgehoben oder zumindest verringert wird.

Rastmittel oder Rastgegenmittel können insbesondere federnd angeordnet sein, so dass sie federnd ineinander greifen können.

Das Rastmittel kann mindestens einen Zahn oder Rastzahn und das Rastgegenmittel mindestens einen, bevorzugt mehrere Zähne oder Rastzähne aufweisen, die so geformt sind, dass das Befestigungsglied in eine Richtung bewegbar und in die andere Richtung nicht bewegbar ist. Als vorteilhaft haben sich sägezahnförmige Rastzähne herausgestellt. Die Rastzähne sind bevorzugt in Befestigungsrichtung, insbesondere in Umfangsrichtung oder in Längsrichtung aufeinanderfolgend angeordnet, insbesondere aneinander gereiht. Durch die aneinander- oder aufeinanderfolgende Anordnung werden die diskreten Rastschritte beim Befestigen erreicht. Prinzipiell reicht es aus, wenn eines aus Rastmittel und Rastgegenmittel einen bzw. einen einzigen Zahn und das andere aus Rastmittel und Rastgegenmittel mehrere Zähne aufweist, insbesondere entsprechend der gewünschten Anzahl an diskreten Rastschritten.

In einer besonders bevorzugten Ausführungsform weist die Ampullenaufnahme eine Nut auf, wobei eine oder beide zueinander weisenden Nutflanken jeweils ein Rastgegenmittel aufweisen. Das Befestigungsglied weist ein Rastmittel auf, das federnd angeordnet ist und gegen die ihr zugeordnete Nutflanke, insbesondere in das Rastgegenmittel gedrückt wird. Bevorzugt ist je Nutflanke ein Rastmittel an dem Befestigungsglied angeordnet.

Die Erfindung wurde anhand mehrerer Ausführungsbeispiele beschrieben. Im Folgenden wird eine Ausführung der Erfindung anhand von Figuren beschrieben. Die hierbei offenbarten Merkmale bilden je einzeln und in Kombination die Erfindung vorteilhaft weiter. Es zeigen:
- Figuren 1-4: Teile einer Nadeleinheit,
- Figuren 5 und 6: die Einzelteile einer Ampullenhalterung und eine bevorzugte Ausführung einer Nadeleinheit,
- Figur 7: eine zusammengesetzte Ampullenhalterung zusammen mit einer darin aufgenommenen Ampulle,
- Figur 8: die Ampullenhalterung aus Figur 7 mit einer daran und einer an der Ampulle angeordneten Nadeleinheit,
- Figur 9: eine bevorzugte Ausführung eines Befestigungsabschnitts der Nadeleinheit,
- Figur 10: eine bevorzugte Ausführung einer Verabreichungsvorrichtung,
- Figuren 11a bis 11d: mehrere Ansichten eines Befestigungsglieds,
- Figur 12: eine Ampullenhalterung mit einer Nadeleinheit und dem Befestigungsglied aus den Figuren 11a bis 11d,
- Figur 13: mehrere Ansichten einer Ampullenaufnahme insbesondere mit einem Befestigungsglied,
- Figur 14: mehrere Ansichten einer Ausführungsform einer Ampullenaufnahme und eines Befestigungsglieds und
- Figur 15: noch eine Ampullenaufnahme und ein Befestigungsglieds.

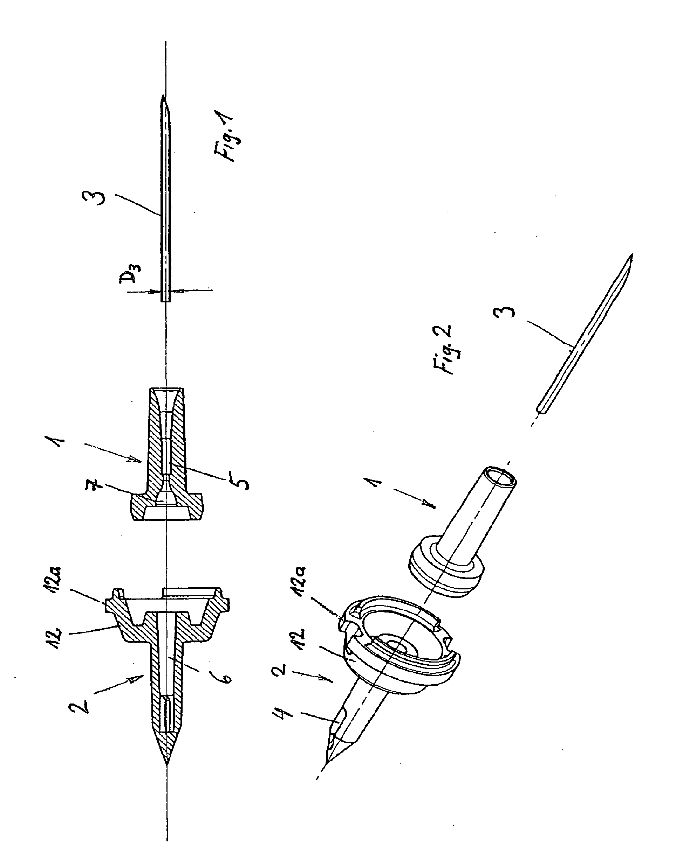

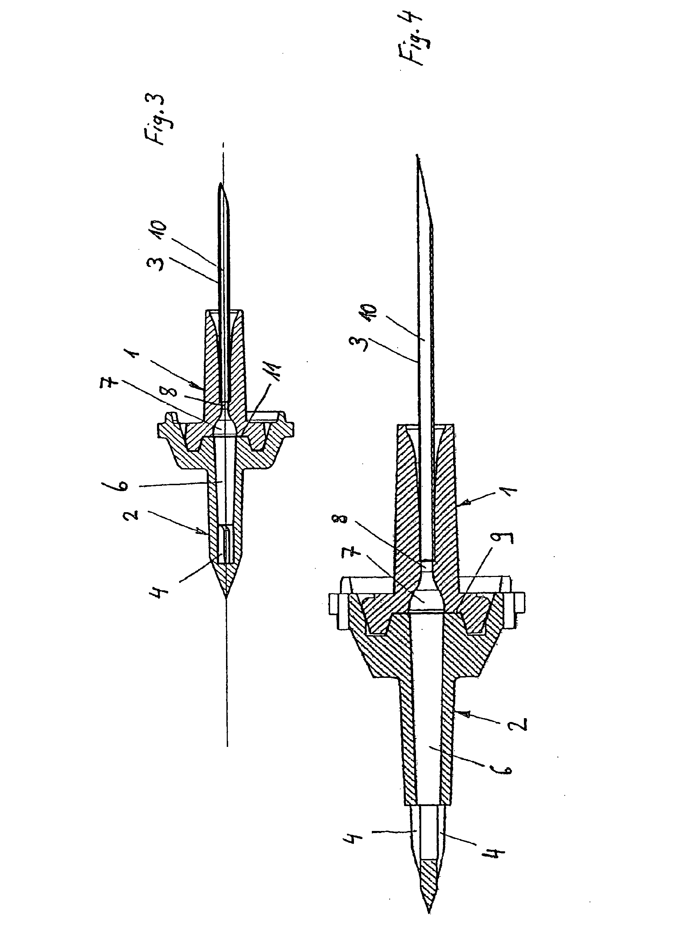

Bezugnehmend auf die Figuren 1-4 werden mehrere Teile einer Nadeleinheit gezeigt, die einen Fluidkanal 6, 7, 8, 10, der auch als Fluidführungskanal bezeichnet werden kann, bilden. Die den Fluidkanal 6, 7, 8, 10 bildenden Teile sind ein Fortsatz 2, der auch als Stechmittel bezeichnet werden kann, ein Nadelträger 1, der auch als Kanülenträger bezeichnet werden kann, und eine Injektionsnadel 3, die auch als Kanülenrohr bezeichnet werden kann.

Die hohlzylindrische Injektionsnadel 3 weist bevorzugt einen konstanten Außendurchmesser D₃ sowie einen konstanten Innendurchmesser auf. An ihrem distalen Ende befindet sich eine Schneidspitze, die im gezeigten Schnitt asymmetrisch ist. Die Injektionsnadel 3 dient zum Einstechen in die Haut eines Patienten und ist mit ihrem proximalen Ende an dem Nadelträger 1 befestigt. Die hierin gezeigte Befestigung besteht darin, dass ein Teil der Injektionsnadel in einer hohlzylindrischen Bohrung 5 aufgenommen ist, deren Innendurchmesser in etwa dem Außendurchmesser der Nadel 3 entspricht. Grundsätzlich könnte die Nadel 3 in dem hohlzylindrischen Abschnitt des Nadelträgers 1 eingepresst oder eingegossen sein, wie z.B. bei der Spritzgussherstellung des Nadelträgers 1. Bevorzugt ist die Nadel in den Nadelträger 1 eingeklebt. Um Spannungsspitzen bei auf die Nadel 3 ausgeübten Querkräften zu verhindern, schließt sich an den Abschnitt 5 ein sich aufweitender, insbesondere trompetenförmig aufweitender Bereich an, der wie hier gezeigt ist, zum distalen Ende des Nadelträgers 1 reicht. Dieser Bereich kann alternativ oder zusätzlich auch dazu dienen, Klebstoff für die Klebeverbindung zwischen dem Nadelträger 1 und der Nadel 3 aufzunehmen. Bevorzugt wird somit, dass die Nadel 3 unlösbar an dem Nadelträger 1 befestigt ist. Die Nadel 3 ist aus einem entsprechend für Injektionsnadeln geeigneten Metall und der Nadelträger 1 bevorzugt aus Kunststoff.

Der Nadelträger 1 weist einen Abschnitt 8 des Fluidkanals auf, der sich an das proximale Ende der Injektionsnadel 3 anschließt. Der Abschnitt 8 kann bevorzugt hohlzylindrisch sein. Insbesondere weist der Abschnitt 8 in etwa den gleichen Fluidführungsquerschnitt, insbesondere den gleichen Innendurchmesser wie die Injektionsnadel 3 auf. Durch diese Gestaltung kann beim Übergang des Fluids von Abschnitt 8 in den Abschnitt 10 der Injektionsnadel 3 die Gefahr einer Wirbelbildung oder turbulenten Strömung verringert werden.

Der Nadelträger 1 weist ferner einen Abschnitt 7 des Fluidführungskanals auf, der einen größeren Fluidführungsquerschnitt aufweist als die Abschnitte 8 und 10 des Fluidführungskanals. Der Abschnitt 7 verjüngt sich in Richtung Injektionsnadel 3, d.h. dass sich der Fluidführungsquerschnitt in Abschnitt 7 in Richtung Nadel 3 verringert. Die Verringerung kann z.B. mit einer konstanten Rate, d.h. einem konstanten Gradienten oder wie hier dargestellt mit einem zunehmenden und/oder abnehmende Gradienten erfolgen. In Richtung Injektionsnadel 3 erfolgt zunächst eine Verringerung des Fluidführungsquerschnitts mit einem zunehmenden Gradienten. Anschließend erfolgt eine Verjüngung des Fluidführungsquerschnitts mit einem abnehmenden Gradienten. Im Längsschnitt kann der Abschnitt 7 des Fluidkanals eine konkave und/oder eine konvexe Wölbung der Kanalwand aufweisen. In Richtung zur Injektionsnadel 3 weist die Wandung des Abschnitts zunächst eine konkave Wölbung und anschließend eine konvexe Wölbung auf Die Übergänge zwischen den einzelnen Abschnitten, wie z.B. der Übergang von konkav auf konvex, kann stetig oder zumindest ohne scharfe Kante ausgestaltet sein. Gleiches gilt für den Übergang des Abschnitts 7 in den Abschnitt 8.

Der Nadelträger weist einen sich in distale Richtung erstreckenden, z.B. zylindrischen Schaft auf, in dem die Nadel 3 angeordnet und befestigt ist.

Der Nadelträger 1 ist mit einer Fügeverbindung 9 mit dem Fortsatz 2 verbunden. Die Fügeverbindung kann kraft-, stoff- oder formschlüssig sein. Insbesondere ist die Fügeverbindung fluiddicht. Eine besonders geeignete Fügeverbindung kann durch Verkleben, Verpressen, Verrasten oder Verschweißen erzielt werden.

Der mit dem Nadelträger 1 verbundene Fortsatz 2 weist einen Abschnitt 6 des Fluidführungskanals 6, 7, 8, 10 auf. Der Abschnitt 6 weitet sich in Richtung Injektionsnadel 3 auf, wie z.B. trichter- oder kegelförmig. Ein Mass für die Aufweitung kann ein Kegelwinkel von ca. 4° sein, um nur ein Beispiel zu nennen. Der Kegelwinkel kann z.B. aus einem Bereich von 1-45° sein, wobei eher geringere Kegelwinkel bevorzugt werden.

Bevorzugt ist, dass der Abschnitt 6 in den Abschnitt 7 mündet, wenn der Nadelträger 1 mit dem Fortsatz 2 verbunden ist. Die Abschnitte 6 und 7 können an der Mündung jeweils einen gleichen Fluidführungsquerschnitt, insbesondere Innendurchmesser, aufweisen.

Der Abschnitt 6 kann z.B. ein Querschnittsverhältnis von seinem größten Fluidführungsquerschnitt zu seinem kleinsten Fluidführungsquerschnitt von 4:1 aufweisen. Als vorteilhaft haben sich Querschnittsverhältnisse von 2:1 bis 10:1 herausgestellt. Das Verhältnis kann auch 2,5:1 betragen.

Das zu verabreichende Fluid wird von der Umgebung des Fortsatzes 2 über eine Öffnung 4 dem Fluidkanal 6, 7, 8, 10 zugeführt. Obwohl grundsätzlich eine Öffnung 4 ausreichen würde, sind in dem Beispiel vorteilhafterweise zwei Öffnungen 4 vorgesehen, die einander gegenüberliegend und seitlich des Fluidkanals 6 angeordnet sind. Ein Teil des Fluidkanals 6 ist somit zwischen den Öffnungen 4 angeordnet. Die Öffnungen 4 haben die Gestalt eines Langlochs, das z.B. zwischen 1 und 4 mm lang sein kann, wie z.B. 3 mm, und eine Breite wie der Durchmesser des zwischen den Öffnungen angeordneten Teils des Fluidkanals 6 aufweisen kann. Bevorzugt kann die Gesamtquerschnittsfläche der Öffnungen 4 größer sein als der kleinste Fluidführungsquerschnitt des Abschnitts 6. Vorteilhaft ist bereits der Querschnitt einer Öffnung 4 größer. Das Verhältnis der Gesamtquerschnittsfläche der Öffnungen 4 und des kleinsten Fluidführungsquerschnitts des Abschnitts 6 kann z.B. 7:1 betragen. Zumindest sollte die Gesamtquerschnittsfläche der Öffnungen 4 genauso groß wie der kleinste Fluidführungsquerschnitt sein. Das Querschnittsverhältnis kann sich z.B. in einem Bereich von 2:1 bis 10:1 bewegen.

Der Fortsatz 2 weist eine rotationssymmetrische Spitze auf, die in diesem Beispiel als Kegel ausgestaltet ist und das distale Ende des Fortsatzes 2 bildet. Die Anordnung der beiden Öffnungen 4 bewirkt, dass das Fluid seitlich, d.h. quer zur Längsachse, zugeführt und anschließend im Fluidführungskanal 6, 7, 8, 10 entlang der Längsachse in Richtung Nadel 3 transportiert wird.

Der Fortsatz 2 weist einen Verbindungsabschnitt 12 auf, mit dem die Einheit aus Nadelträger 1, Fortsatz 2 und Nadel 3 gegebenenfalls mittelbar oder unmittelbar mit einem Teil der Injektionsvorrichtung verbindbar ist. Alternativ könnte der Nadelträger 1 den Abschnitt 12 aufweisen.

Das proximale Ende des Nadelträgers 1 weist im Bereich des Fluidkanals 7 eine Vertiefung auf, die von einem ringförmigen Vorsprung umgeben wird. Der ringförmige Vorsprung ist in diesem Beispiel konzentrisch mit der Längsachse der Nadeleinheit 100. Der Fortsatz 2 weist an seinem distalen Ende eine Form auf, die in etwa einer Negativform der proximalen Stirnseite des Nadelträgers 1 entspricht. Insbesondere weist die distale Stirnseite des Fortsatzes 2 eine Ringnut, in die beim Zusammenfügen des Fortsatzes 2 mit dem Nadelträger 1 der ringförmige Vorsprung angeordnet wird, und einen kegelstumpfförmigen Vorsprung, der beim Zusammenfügen in die Vertiefung des Nadelträgers 1 eingreift, auf. Hierdurch wird bewirkt, dass eine besonders vorteilhafte Fügeverbindung 9, die insbesondere auch fluiddicht ist, erzielt wird. Durch das Ineinandergreifen der beiden Stirnseiten wird die für eine Fügeverbindung 9 nutzbare Oberfläche vergrößert.

Bei der Injektionsnadel 3 kann es sich um Nadeln mit einer großen Bandbreite von Durchmessern, Wandstärken und Längen handeln. Z.B. kann eine 23 Gauge Nadel genauso vorgesehen werden wie eine 31 Gauge Nadel. Die injizierbare Länge der Injektionsnadel 3 kann z.B. für eine subkutane oder transkutane Injektion vorgesehen sein.

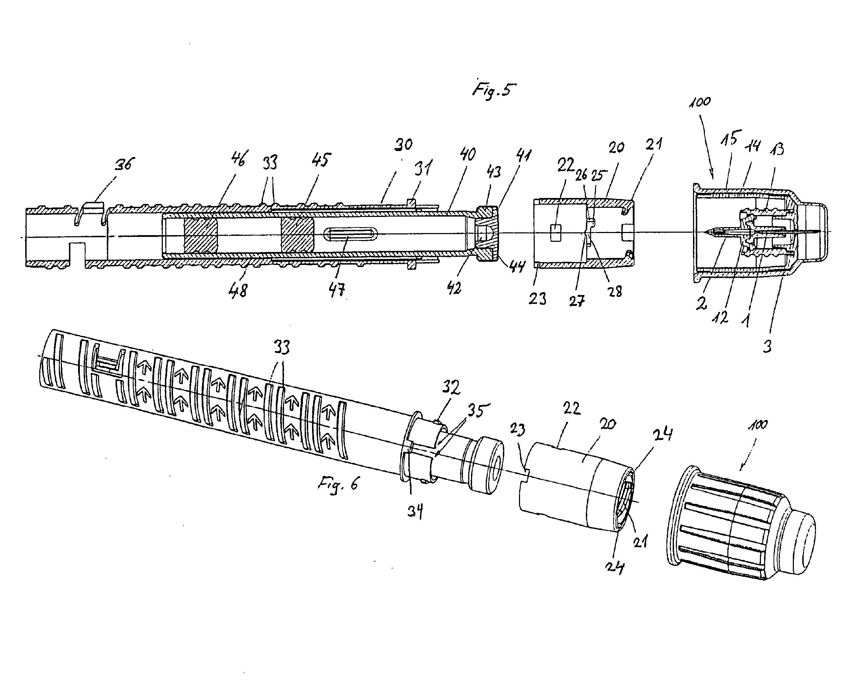

Insbesondere wird in den Figuren 5 und 6 eine vollständige Nadeleinheit 100 gezeigt. Die Einheit aus Nadelträger 1, Fortsatz 2 und Injektionsnadel 3 ist über den Verbindungsabschnitt 12 mit einem Befestigungsabschnitt 13 verbunden. Der hülsenförmige Befestigungsabschnitt 13 dient dazu, mit einem Befestigungsglied 20 verbunden zu werden und weist hierzu ein Gewinde, insbesondere ein Außengewinde auf. Die den Fluidkanal 6, 7, 8, 10 bildende Einheit ist axial fest mit dem Befestigungsabschnitt 13 verbunden. Je nach Anwendung kann die Einheit 1, 2, 3 relativ zu dem Befestigungsabschnitt 13 drehbar oder drehfest sein. Die Einheit 1, 2, 3 weist einen Verbindungsvorsprung 12a auf, der in eine am inneren Umfang des Befestigungsabschnitts 13 gebildete Ringnut 13c eingreift. Der Befestigungsabschnitt 13 ist bevorzugt dreh- und axial fest mit einem Gehäuse 14 verbunden. Grundsätzlich könnten der Befestigungsabschnitt 13 und das Gehäuse 14 auch einteilig gebildet sein, wobei die Mehrteiligkeit Vorteile bei der Herstellung der Nadeleinheit 100 hat. Das hülsenförmige Gehäuse 14 umgibt umfangsseitig sowohl den Befestigungsabschnitt 13 als auch den Fortsatz 2 und dessen Spitze. Das Gehäuse 14 erstreckt sich in proximale Richtung zumindest bis zur Spitze oder wie hier gezeigt bis über die Spitze hinaus, so dass die Spitze des Fortsatzes 2 nicht über das proximale Ende des Gehäuses 14 hervorsteht. Durch den Überstand des Gehäuses 14 über die Spitze des Fortsatzes 2 wird die Gefahr eines versehentlichen Stechens durch die Spitze 2 verringert. Zwischen dem Befestigungsabschnitt 13 und dem Gehäuse 14 wird ein Ringspalt gebildet. Das Gehäuse 14, der Befestigungsabschnitt 13 und die Einheit 1, 2, 3 sind konzentrisch zueinander angeordnet.

Das Gehäuse 14 weist an seinem distalen Ende eine Stirnfläche mit einer Öffnung auf, durch die die Injektionsnadel 3 mit einer bestimmten Länge hervortritt. Diese Länge entspricht im Wesentlichen der Injektionstiefe der Injektionsnadel 3, da das distale Ende des Gehäuses eine Anschlagfläche für den Körper des Patienten bildet. Das Gehäuse 14 kann z.B. aus Platzgründen eine Aussparung für den Nadelträger 1 aufweisen.

Die Nadeleinheit 100 weist ferner eine Verpackungshülse 15 auf, die in der in den Figuren 5 und 6 gezeigten Positionen über den äußeren Umfang des Gehäuses 14 konzentrisch angeordnet ist. Bevorzugt wird die Verpackungshülse 15 von dem Gehäuse 14 insbesondere reibschlüssig gehalten. Die Verpackungshülse 15 umgibt das Gehäuse 14 bevorzugt vollständig über den Umfang und auch über dessen Länge. Bevorzugt steht die Verpackungshülse 15 proximal über das proximale Ende des Gehäuses 14 zumindest ein Stück weit über. Die am proximalen Ende der Verpackungshülse 15 gebildete Öffnung, durch die das Gehäuse 14 mit der Einheit 1, 2, 3 einführbar oder herausziehbar ist, kann im Auslieferungszustand der Nadeleinheit 100 mit einer sog. Peelfolie verschlossen sein, um den Inhalt der Verpackungshülse 15 steril zu halten oder zumindest vor Verschmutzung zu schützen. Die Verpackungshülse 15 steht distal über das distale Ende der Nadel 3 über und ist dort stirnseitig verschlossen. Die Verpackungshülse 15 bildet insoweit einen geschlossenen Topf, der an seinem proximalen Ende eine Öffnung aufweist. Die Verpackungshülse 15 weist an ihrem äußeren Umfang eine Struktur auf, welche es einem Verwender der Vorrichtung besser ermöglicht, ein Drehmoment um die Längsachse auf die Verpackungshülse 15 aufzubringen. Da die Verpackungshülse 15 in ihrem auf dem Gehäuse 14 vollständig aufgesetzten Zustand zumindest reibschlüssig bevorzugt alternativ oder zusätzlich formschlüssig drehfest mit dem Gehäuse 14 verbunden ist, dreht sich das Gehäuse 14 zusammen mit dem Befestigungsabschnitt 13 mit der Verpackungshülse 15 mit, wenn ein Drehmoment auf die Verpackungshülse 15 aufgebracht wird.

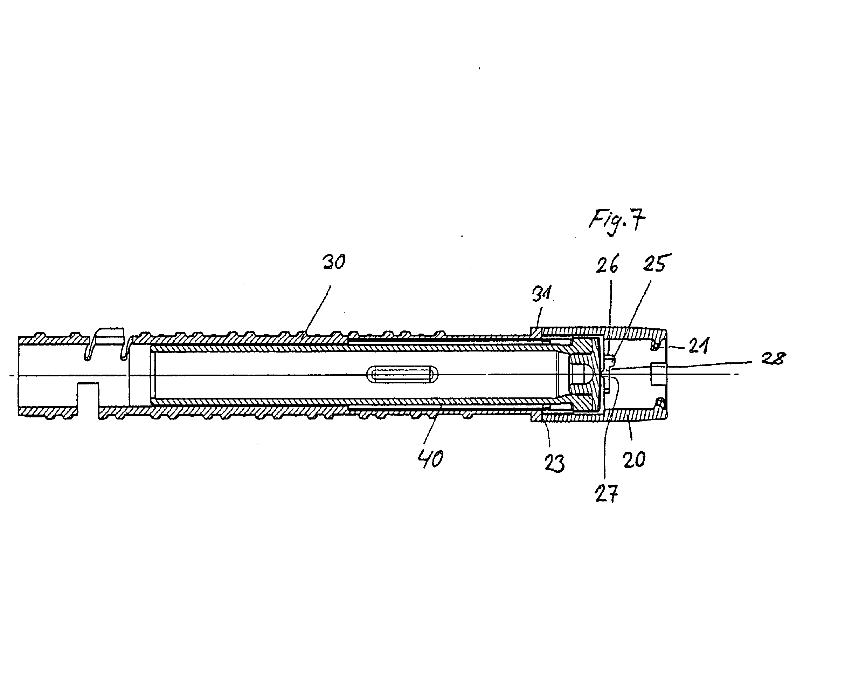

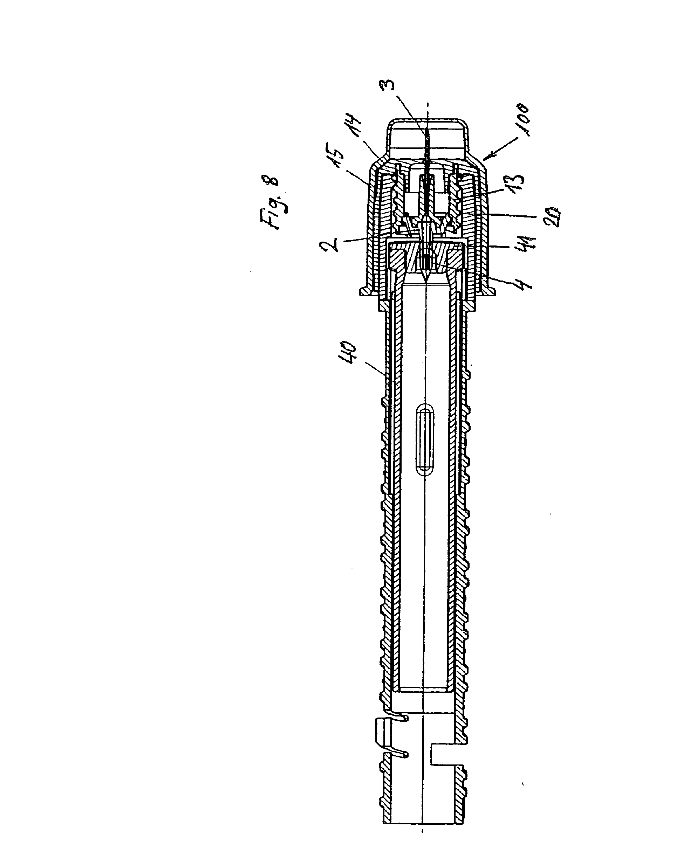

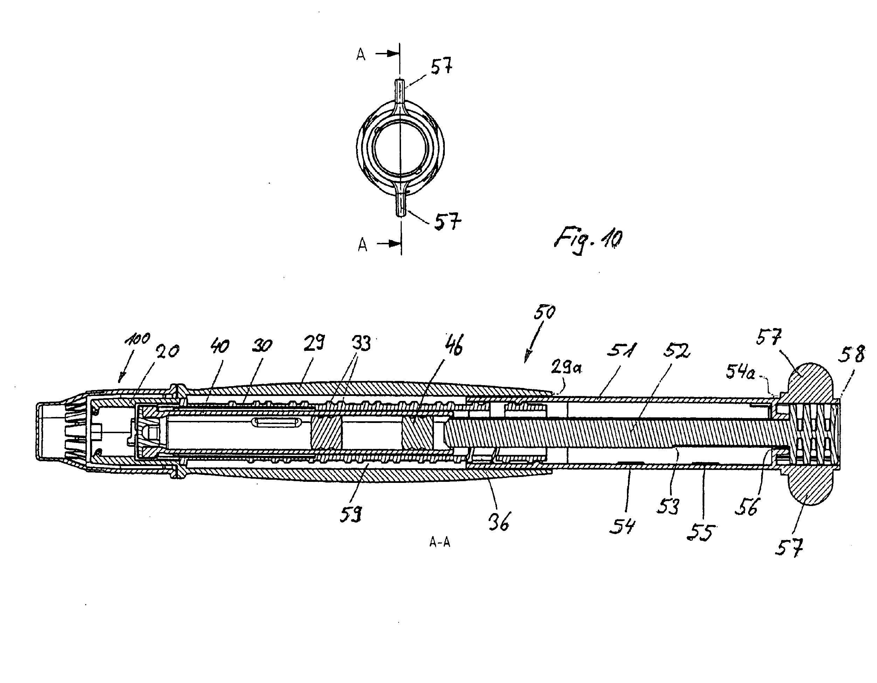

Die Nadeleinheit 100 kann an eine Injektionsvorrichtung 50 angebracht werden (Figur 10). Teile der Injektionsvorrichtung, nämlich eine Ampullenhalterung 30 und ein Befestigungsglied 20, werden zusammen mit einer Ampulle 40 in den Figuren 5 und 6 vereinzelt, in Figur 7 zusammengesetzt und in Figur 8 zusammengesetzt mit einer aufgesetzten Nadeleinheit 100 gezeigt.

Bei der Ampulle 40, wie sie z.B. in den Figuren 5 und 6 gezeigt wird, handelt es sich um eine sog. Zweikammerampulle, die auch als Zweikammerkarpule bezeichnet werden kann, deren Besonderheit es ist, dass ein zu verabreichendes Produkt unmittelbar vor der Verabreichung aus zwei Komponenten zusammengemischt wird, die zur Lagerung in zwei verschiedenen Kammern enthalten sind. Z.B. kann die distale Kammer, d.h. die Kammer, die zwischen einem Verschluss 41, 42, 43, 44 und einem ersten Kolben 45 angeordnet ist, mit einem Feststoff wie z.B. einem körnigen oder pulverförmigen Material teilweise oder ganz gefüllt sein. Proximal des ersten Kolbens 45 befindet sich eine zweite Kammer, die zwischen dem ersten Kolben 45 und einem zweiten Kolben 46 angeordnet ist. In dieser Kammer kann sich z.B. ein flüssiger Bestandteil des Medikaments befinden. Beide Kolben 45, 46 liegen dichtend an einem hohlzylindrischen Gehäuseabschnitt 48 der Ampulle 40 an und können entlang dem Gehäuseabschnitt 48 verschoben werden.

Die Ampulle 40 weist im Bereich des Gehäuseabschnitts einen sog. Bypass 47 auf, der nach aussen eine Auswölbung bildet. Der Bypass 47 ist axial länger als der erste Kolben 45.

Zum Abmischen wird distal gerichtet ein Druck auf den zweiten Kolben 46 ausgeübt, der über das flüssige Produkt in der Kammer zwischen dem ersten Kolben 45 und dem Kolben 46 auf den Kolben 45 übertragen wird. Wenn der zweite Kolben 46 in distale Richtung verschoben wird, wird somit auch der ersten Kolben 45 verschoben und zwar bis in den Bereich des Bypasses 47. Durch die Aufwölbung des Bypasses 47 kann, sofern sich der Kolben 45 vollständig im Bereich des Bypasses 47 befindet, das flüssige Produkt an dem Kolben 45 vorbei fließen in die distale Kammer und sich mit dem darin befindlichen Produktbestandteil mischen. Das Mischen kann vom Verwender durch Schütteln unterstützt werden. Das flüssige Produkt ist vollständig aus der proximalen Kammer beseitigt, wenn der zweite Kolben 46 an den ersten Kolben 45 anschlägt. Das Gemisch aus den beiden Produktbestandteilen kann eine homogene oder heterogene Mischung bilden. Die heterogene Mischung eines flüssigen Bestandteils mit Festkörperpartikeln wird allgemein als Dispersion, im Speziellen als Suspension bezeichnet. Das zu verabreichende Produkt kann dann über den Fluidkanal 6, 7, 8, 10 der Nadeleinheit 100, deren Öffnungen 4 sich innerhalb der Ampulle wie z.B. im Bereich 42 befinden, ausgeschüttet werden, indem die Kolben 45 und 46 in distale Richtung entlang des hohlzylindrischen Gehäuseabschnitts 48 verschoben werden.

Die Ampulle 40 weist einen Verschluss 41, 42, 43, 44 auf, der ein von dem Fortsatz 2 durchstechbares Septum 41 aufweist. Solche Septen können aus Gummi oder Kautschuk oder einem anderen geeigneten Kunststoff sein.

Das Septum 41 weist einen scheibenförmigen Abschnitt und einen sich daran in proximale Richtung anschließenden hohlzylindrischen Abschnitt auf. Der hohlzylindrische Abschnitt bildet in seinem Inneren eine Ausnehmung, in der in bevorzugten Ausführungen die Öffnungen 4 der vollständig aufgesetzten Nadeleinheit 100 platziert sind. Die Außenseite des hohlzylindrischen Abschnitts des Septums 41 zentriert das Septum 41 am inneren Umfang der Ampulle 40, insbesondere am distalen Ende der Ampulle 40. Am distalen Ende der Ampulle 40 ist ein ringförmig umlaufender Wulst gebildet, der radial nach außen ragt und allgemein als Abragung bezeichnet werden kann. Der Wulst 43 steht radial über den äußeren Umfang des hohlzylindrischen Abschnitts 48 der Ampulle 40. Mit anderen Worten weist die Ampulle 40 an der Stelle des Wulsts 43 einen größeren Außendurchmesser auf als im Bereich des hohlzylindrischen Abschnitts 48. Der Außendurchmesser des Wulsts 43 bildet in diesem Beispiel auch den größten Außendurchmesser der Ampulle 40. Der Wulst 43 ist einteilig mit dem hohlzylindrischen Abschnitt 48 verbunden. Dieser Teil der Ampulle kann aus Kunststoff oder aus Glas gebildet sein. Der scheibenförmige Teil des Septums 41 weist in etwa den gleichen Außendurchmesser auf wie der Wulst 43. Septum 41 und Wulst 43 werden zusammengehalten durch ein Umformteil 44, das z.B. aus einem Metallblech gefertigt sein kann. Dieses Teil kann auch als Krone bezeichnet werden. Das Umformteil 44 umgreift das Septum 41 und den Wulst 43 über den Umfang und sowohl distal des Septums 41 als auch proximal des Wulsts 43. Durch das Umformteil 44 werden Wulst 43 und Septum 41 axial fest verbunden, d.h. fluiddicht zusammengehalten. An der distalen Stirnseite weist das Umformteil 44 eine Öffnung auf, die z.B. kreisförmig sein kann und durch die der Fortsatz 2 durch das Septum 41 in das Innere der bevorzugt bereits abgemischten Ampulle 40 eingeführt werden kann.

Die Ampulle 40 weist zwischen dem hohlzylindrischen Teil 48 und dem Wulst 43 einen gegenüber dem Außendurchmesser des Abschnitts 48 eingeschnürten Hals auf, so dass das Umformteil 44 noch besser, insbesondere weiter nach innen, um das proximale Ende des Wulsts 43 umformbar ist. Das proximale Ende der Ampulle 40 ist offen, so dass eine Kolbenstange 52 (Figur 10) in die Ampulle 40 und auf den ersten Kolben 46 schiebbar ist.

An der Ampullenaufnahme 30 kann das Befestigungsglied 20 befestigt sein oder werden. Befestigungsglied 20 und Ampullenaufnahme 30 werden vorzugsweise als Ampullenhalterung bezeichnet. Das Befestigungsglied 20 ist hülsenförmig und dreh- und axialfest mit der Ampullenaufnahme 30 verbunden, insbesondere verrastet. Die Drehfestigkeit wird durch den Eingriff der Aussparung 23 in den Nocken 34 erzielt. Die axial feste Anordnung des Befestigungsglieds 20 an der Ampullenaufnahme 30 wird durch das Einrasten der Nocken 32 in die Fenster 22 erzielt.

Wie aus den Figuren 5 und 6 erkennbar ist, ist die Ampulle 40 über eine distale Öffnung der hülsenförmigen Ampullenaufnahme 30 in diese eingeführt. Der Außendurchmesser des Verschlusses 41, 42, 43, 44 ist größer als der Innendurchmesser der Ampullenaufnahme 30, so dass der Verschluss am distalen Ende der Ampullenaufnahme 30 anliegt. Wie insbesondere aus Figur 7 erkennbar ist, ist der Verschluss der Ampulle 40 zwischen der Ampullenaufnahme 30 und dem Befestigungsglied 20 axial eingefasst und somit axial fixiert. Die Einfassung kann beispielsweise ein geringes axiales Spiel der Ampulle 40 zulassen. Bevorzugt ist es jedoch, dass die Einfassung dergestalt ist, dass das axiale Spiel weiter verringert wird. Hierzu weist das distale Ende der Ampullenaufnahme 30 wenigstens eine Abragung, in diesem Fall vier Abragungen 35 auf, die sich in distale Richtung erstrecken und an denen der Verschluss 41, 42, 43, 44 anliegt. Die mindestens eine Abragung 35 kann starr, wie hier gezeigt, oder federnd an der Ampullenhalterung, insbesondere an der Ampullenaufnahme, angeordnet sein. Die federnde Anordnung hat zudem den Vorteil, dass ein axiales Spiel so gut wie nicht mehr vorhanden ist. Die federnde Anordnung kann dadurch erzielt werden, dass proximal der mindestens einen Abragung 35 ein sich in Umfangsrichtung erstreckender Schlitz vorgesehen ist, so dass der sich zwischen Schlitz und Abragung 35 befindliche Steg in Axialrichtung federn kann.

Der Verschluss 41, 42, 43, 44 kann von der distalen Seite her durch eine am inneren Umfang des Befestigungsglieds 20 gebildete Schulter eingefasst werden. Die Schulter kann teilweise oder vollständig über den Umfang umlaufen. Bevorzugt ist eine sich von der inneren Umfangsfläche nach innen erstreckende Abragung 28 vorgesehen, entweder alternativ oder zusätzlich zu der Schulter und kann als Einfassung für den Verschluss 41, 42, 43, 44 dienen. Die Abragung 28 weist an ihrer zum Verschluss 41, 42, 43, 44 hinweisenden Seite eine Abragung, insbesondere einen Nocken 27 auf, der gegen die distale Stirnfläche des Verschlusses drückt, insbesondere gegen das Umformteil 44. Durch die punktuelle Belastung des Nockens 27 kann sich ein Teil des Verschlusses, wie z.B. das Umformteil 44 elastisch oder plastisch verformen, wodurch eine sichere, spielarme Einfassung des Verschlusses 41, 42, 43, 44 zwischen der Ampullenaufnahme 30 und dem Befestigungsglied 20 erreicht wird. Der Nocken 27 kann starr oder in Längsrichtung federnd angeordnet sein.

Wie aus den Figuren 5-7 erkennbar ist, weist die Ampullenaufnahme 30 an ihrem äußeren Umfang ein Außengewinde 33 auf, mit dem die Ampullenhalterung in ein Gehäuse 51 der Injektionsvorrichtung 50 (Figur 10) einschraubbar ist, wobei das Gehäuse 51 ein entsprechendes Innengewinde für das Außengewinde 33 der Ampullenhalterung aufweist. Um dem Verwender die intuitive Handhabung der Vorrichtung 50 zu erleichtern, kann an der Ampullenhalterung 20, 30, insbesondere am Umfang der Ampullenaufnahme 30 mindestens ein Pfeil angeordnet sein, der in die Drehrichtung zum Abmischen der Vorrichtung weist. Bevorzugt sind eine Vielzahl von Pfeilen über die Länge der Ampullenaufnahme 30 verteilt. Die Pfeile können z.B. aufgedruckt oder vorzugsweise als Erhebungen oder Vertiefungen, die insbesondere bereits bei der Spritzgussformung der Ampullenaufnahme 30 geformt werden, ausgebildet sein. Der mindestens eine Pfeil kann zwischen benachbarten Gewindegängen 33 angeordnet sein.

Die Ampullenhalterung, insbesondere die Ampullenaufnahme 30 weist ferner ein in radiale Richtung federnd gelagertes Rastglied 36 auf. Die Federung wird mittels eines sich in Umfangsrichtung erstreckenden Arms, der an der Ampullenhalterung 30 gebildet ist und an dem das Rastglied 36 gebildet ist, bewirkt. Die Funktion dieses Rastglieds 36, insbesondere Nockens, wird später mit Bezug auf Figur 10 beschrieben.

Das Befestigungsglied 20 weist ferner ein ein- oder mehrgängiges Gewinde, in diesem Beispiel in der Gestalt von zwei Gewindeabschnitten 21 auf, die es ermöglichen, dass die Nadeleinheit 100 mit dem auf dem Befestigungsabschnitt 13 befindlichen Außengewinde auf die Injektionsvorrichtung aufgeschraubt werden kann. Durch die Axialbewegung des Fortsatzes 2 beim Aufschrauben durchsticht die Spitze des Fortsatzes 2 das Septum 41. Das für das Aufschrauben erforderliche Drehmoment wird über die Verpackungshülse 15 oder das Gehäuse 14 aufgebracht. Nachdem die Nadeleinheit 100 vollständig aufgeschraubt ist, nimmt sie die in Figur 8 gezeigte Position ein. Die Öffnungen hier befinden sich nun innerhalb der Ampulle 40, so dass das distale Ende der Injektionsnadel 3 fluidisch mit dem Innern der Ampulle 40 verbunden ist. Ein distaler Abschnitt des Befestigungsglieds 20 befindet sich nun im Ringspalt zwischen dem Befestigungsabschnitt 13 und dem Gehäuse 14.

Sofern die Einheit 1, 2, 3 relativ zum Befestigungsabschnitt 13 drehbar angeordnet ist, besteht die Möglichkeit, dass der Fortsatz mit einer reinen Axialbewegung in das Septum 41 einsticht. Dies kann Vorteile hinsichtlich der Abdichtung zwischen Septum und Fortsatz ergeben. Bei einer relativ zum Befestigungsabschnitt 13 drehfest angeordneten Einheit 1, 2, 3 wird das Septum vom Fortsatz 2 mittels einer kombinierten Dreh- und Axialbewegung durchstochen, was Vorteile bietet hinsichtlich des für das Aufschrauben benötigten Drehmoments.

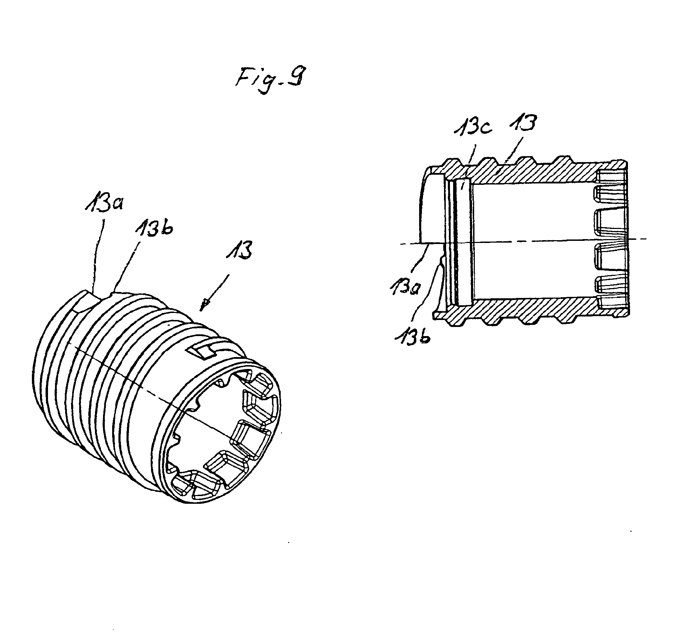

Wie aus den Figuren 5 und 9 erkennbar ist, weisen das Befestigungsglied 20 einen in Umfangsrichtung wirkenden Anschlag 26 und der Befestigungsabschnitt 13 einen ebenfalls in Umfangsrichtung wirkenden Anschlag 13a auf. Es können auch mehrere, wie in diesem Beispiel zwei solche Anschläge jeweils am Befestigungsglied 20 als auch am Befestigungsabschnitt 13 angeordnet sein.

Die Anschläge 26 und 13a bilden ein Anschlagpaar und sind so positioniert, dass sie in der vollständig aufgeschraubten Position der Nadeleinheit 100 (Figur 8) in einen Drehanschlag geraten, wodurch ein Weiterdrehen auf eine sichere Weise verhindert wird. Der Anschlag 13a ist am proximalen Ende des Befestigungsabschnitts 13 gebildet. Der Anschlag 26 ist an der Abragung 28 des Befestigungsglieds 20 gebildet. Optional könnte auch ein Axialanschlag statt eines Drehanschlags vorgesehen sein, wobei der Drehanschlag gegenüber einem Axialanschlag den Vorteil hat, dass die in Anschlag geratenen Teile weniger belastet werden. Denn durch die von der Gewindesteigung erzeugte Übersetzung ist mit einem bestimmten Drehmoment in Axialrichtung eine deutlich höhere Kraft erzielbar als in Umfangsrichtung.

Insbesondere kann das Befestigungsglied 20 eine oder mehrere Abragungen 24 oder 25 aufweisen, die kurz vor dem Erreichen des Drehanschlags mit der Nadeleinheit 100 verrasten, so dass eine Rückdrehung der Nadeleinheit 100 in entgegen gesetzte Richtung nur mit einem erhöhten Drehmoment oder gar nicht, d.h. nur durch Zerstörung eines Bauteils oder eines Teils eines Bauteils möglich ist. Alternativ können statt Abragungen 24, 25 Ausnehmungen vorgesehen sein, die dem gleichen Zweck dienen. Durch das Verrasten beim Aufschrauben erhält der Verwender zum einen ein taktiles Signal, das ihm anzeigt, dass die Nadeleinheit 100 nun vollständig aufgeschraubt ist. Zum andern verhindert es eine unbeabsichtigte Rückdrehung, so dass die Gefahr einer Fehlanwendung verringert werden kann. Sofern als Rückdrehsicherung Ausnehmungen oder Nocken 24 vorgesehen sind, greifen diese in das Gehäuse 14 der Nadeleinheit 100 ein, insbesondere in dessen im Ringspalt gebildete Stirnseite. Die Stirnseite kann hierzu mindestens eine Abragung, wie z.B. Rippen oder einen Nocken oder eine Ausnehmung aufweisen, die kurz vor oder beim Erreichen des Drehanschlags überfahren wird.

Alternativ oder zusätzlich kann eine Abragung 25 in der Gestalt eines in distale Richtung ragenden Nockens vorgesehen sein, der kurz vor oder beim Erreichen des Drehanschlags eine Abragung 13b, insbesondere einen Nocken, überfährt. Die Abragung 13b ist an der proximalen Stirnseite des Befestigungsabschnitts 13 und in Umfangsrichtung versetzt vor dem Anschlag 13a angeordnet. Der Abstand zwischen der Abragung 13b und dem Anschlag 13a kann insbesondere in etwa der in Umfangsrichtung gemessenen Breite der Abragung 25 entsprechen, wodurch erreicht wird, dass der Nocken 25 in einem vollständig aufgeschraubten Zustand zwischen dem Anschlag 13a und der Abragung 13b gehalten ist.

Eine Abragung 13b, die eine Schraubbewegung entgegen der Aufschraubrichtung zulässt, kann in Umfangrichtung beidseits abgeflachte Flanken aufweisen. Eine Abragung 13b, die verhindern soll, dass eine Drehung entgegen der Aufschraubrichtung möglich ist, kann beispielsweise die Form eines Sägezahns aufweisen, der ein Überfahren der Abragung 13b erlaubt aber eine Rückdrehung durch die steile Fläche des Sägezahns verhindert. Gleiches gilt selbstverständlich für die Verrastung der Ausnehmungen oder Abragungen 24 mit den Ausnehmungen oder Abragungen an dem Gehäuse 14.

Die Abragung 13b ist auf der als schraubenförmige Bahn ausgestalteten Stirnseite des Befestigungsabschnitts angeordnet. Die Schraubenform weist in etwa die Steigung des Gewindes des Befestigungsabschnitts 13 auf.

Eine bevorzugte Ausführungsform einer Injektionsvorrichtung bei der die beschriebene Erfindung Anwendung finden kann, ist in Figur 10 gezeigt. Bei der Vorrichtung handelt es sich um eine Abmisch- und Ausschüttvorrichtung 50, die im Folgenden der Einfachheit halber nur als Injektionsvorrichtung bezeichnet wird. Die Injektionsvorrichtung 50 weist ein Gehäuse 51 auf mit einem Innengewinde, in welches die in Figur 7 gezeigte Ampullenhalterung 20, 30, welche ein Außengewinde 33 aufweist, einschraubbar ist. Innerhalb des hülsenförmigen Gehäuses 51 ist eine Kolbenstange 52 angeordnet, die mit einem Injektionsknopf 58 verbunden ist. Der Injektionsknopf 58 ist in einem Ausgangszustand der Injektionsvorrichtung 50, d.h. vor einer Abmischung der Produktbestandteile der Zweikammerampulle 40 auf seiner axialen Länge insbesondere vollständig von dem Gehäuse 51 umgeben. An dem Gehäuse 51 sind zwei radial nach aussen weisende Flügel 57 angeordnet. Durch die plättchenförmigen Flügel wird dem Verwender der Vorrichtung das Halten und das Aufbringen eines notwendigen Drehmoments beim Abmischen erleichtert. Die Flügel 57 dienen somit als Greifhilfe und verbessern vorzugsweise im Zusammenspiel mit einem Greifabschnitt 29 die intuitiv richtige Handhabung. Die Flügel 57 befinden sich am proximalen Ende der Vorrichtung 50.

Die Injektionsvorrichtung kann in einer bevorzugten Ausführungsform eine äußere Hülse 29, die als Greifabschnitt dient, aufweisen, die der Verwender der Vorrichtung 50 mit einer Hand umgreifen kann, während er mit der anderen Hand an den Flügeln 57 das Gehäuse 51 auf die Ampullenhalterung, insbesondere in den zwischen der äußeren Hülse 29 und der Ampullenhalterung 30 gebildeten Ringspalt schraubt. Hierdurch wird der Vorteil erzielt, dass Patienten, die über eingeschränkte motorische Fähigkeiten verfügen, ein sicheres Abmischen der Produktbestandteile erlaubt wird. Am Ende der Abmischsequenz und gegebenenfalls einer Primesequenz geraten der am proximalen Ende der äußeren Hülse 29 gebildete Anschlag 29a und der vom Gehäuse 51 im Bereich des distalen Endes gebildete Anschlag 54a in einen Dreh- oder Axialanschlag, so dass dem Benutzer z.B. hierdurch angezeigt wird, dass der Abmischvorgang und Primevorgang beendet sind.

In einer alternativen, ebenfalls bevorzugten Ausführung kommt die Injektionsvorrichtung 50 ohne die äußere Hülse 29 aus. Der Verwender der Injektionsvorrichtung kann zur Aufbringung des Drehmoments die Verpackungshülse 15 oder das Gehäuse 14 mit der einen Hand und mit der anderen Hand das Gehäuse 51 oder gegebenenfalls daran befestigte Flügel 57 umgreifen. Die Abmischung funktioniert analog zur Ausführung mit einem hülsenförmigen Greifabschnitt 29, wobei das Ende des Abmischvorgangs z.B. durch den Dreh- oder Axialanschlag des distalen Endes des Gehäuses 51 an den in Figur 5 mit dem Bezugszeichen 31 bezeichneten ringförmig umlaufenden Kragen der Ampullenhalterung 20, 30 angezeigt wird. Diese Variante ist auch bei der Ausführung mit einer äußeren Hülse 29 möglich. Aufgrund der Drehanschläge 26, 13a zwischen Nadeleinheit 100 und Ampullenhalterung 20, 30 kann eine Überlastung der am Anschlag beteiligten Bauteile sicher verhindert werden, auch wenn das Drehmoment zum Abmischen über die Nadeleinheit 100 läuft.

Für beide Ausführungsformen einer Injektionsvorrichtung 50 gilt, dass beim Ineinanderschrauben der Ampullenaufnahme 30 und des Gehäuses 51 beim Abmischen der Produktbestandteile die Kolbenstange 52 in einen Anschlag mit dem Kolben 46 gerät, wodurch sich das Gehäuse 51 relativ zu der Kolbenstange 52 in distale Richtung bewegt. Die Kolbenstange 52 bleibt hierbei axial fest relativ zur Ampulle 40 aufgrund der Haftreibung zwischen dem/den Kolben 45, 46 und der Ampullenwand. Die Kolbenstange 52 weist eine Aussparung auf, an deren distalen Ende 53 ein Anschlag 53 gebildet ist. Während des Einschraubens bewegt sich der von dem Gehäuse 51 gebildete Axialanschlag 56 in Richtung Anschlag 53. Der Injektionsknopf 58 tritt bei dieser Bewegung proximal aus dem Gehäuse 51 hervor. Sobald der Anschlag 56 an den Anschlag 53 anstößt, wird die Kolbenstange 52 mitgenommen, so dass die Kolbenstange 52 die Axialbewegung des Gehäuses 51 in distale Richtung relativ zu der Ampullenhalterung 30 und zur Ampulle 40 mitmacht. Die Kolbenstange 52 kann nun den Kolben 46 in Richtung distal verschieben, wodurch die Abmischsequenz durchgeführt wird, wie weiter oben beschrieben wurde. Am Ende des Abmischens kann das Rastglied 36 in ein Rastelement 54 eingreifen oder dieses überfahren, wodurch dem Verwender das Ende des Abmischvorgangs angezeigt werden kann. Der Verwender schüttelt nun die Vorrichtung 50, so dass sich die Produktbestandteile vermischen. Anschließend kann er die Vorrichtung primen, um eventuell in der Ampulle 40 noch enthaltene Luft aus der Ampulle 40 zu entfernen. Hierzu kann der Verwender die Schraubbewegung des Gehäuses 51 relativ zu der Ampullenhalterung 30 fortsetzen, wobei dann beide Kolben verschoben werden und das Volumen im Produktbehältnis verkleinert wird, wodurch die Luft aus dem Produktbehältnis 40 über den Fluidkanal 6, 7, 8, 10 der Nadeleinheit 100 ausgestoßen wird. Am Ende der Primesequenz kann das Rastglied 36 in ein weiteres ebenfalls am inneren Umfang des Gehäuses 51 gebildetes Rastelement 55 einrasten, wodurch dem Verwender das Ende der Primesequenz angezeigt wird. Die Vorrichtung 50 ist nun bereit für eine Produktausschüttung, die nach dem Einstechen der Nadel 3 in eine gewünschte Körperstelle durch Drücken des Injektionsknopfs 58 in distale Richtung relativ zum Gehäuse 51 und zur Ampulle 40 bewirkt wird.

Die Rastelemente 54 und 55 können so gebildet sein, dass bei einer Wechselwirkung, insbesondere beim Überfahren des Rastglieds 36 über die Rastelemente 54 und 55 ein akustisches oder taktiles Signal erzeugt wird. Ferner ist durch eine in Umfangrichtung asymmetrische Ausbildung der Flanken am Rastglied 36 realisierbar, dass nachdem das Rastglied 36 die Rastelemente 54 oder 55 erreicht hat, ein Weiterdrehen in eine der Abmisch- oder Primebewegung entgegengesetzte Richtung nicht mehr möglich ist.

Bei der Ausführungsform mit einer äußeren Hülse 29 kann diese dreh- und axial fest mit der Ampullenaufnahme 30 verbunden sein, wie z.B. durch eine Rastverbindung oder durch eine einteilige Ausbildung mit der Ampullenhalterung 30 oder dem Befestigungsglied 20. Die äußere Hülse 29 kann sich von dem Befestigungsglied 20 soweit in proximale Richtung erstrecken, dass ihr proximales Ende proximal über das proximale Ende der Ampulle 40 und insbesondere auch über das proximale Ende der Produktbehältnisaufnahme 30 ragt.

Die in Figur 10 gezeigte Ausführung hat den Vorteil, dass der Verwender der Vorrichtung 50 kein Gewinde sieht, was vor allem bei Menschen, die Technik skeptisch gegenüber stehen, von Vorteil ist. Z.B. kann auf der Außenseite des Gehäuses 51 eine Farbkodierung angegeben sein, aus der zusätzlich optisch ersichtlich ist, ob die Vorrichtung sich beim Abmischen oder beim Primen befindet oder ob die Vorrichtung fertig für eine Produktausschüttung ist. Z.B. kann eine erste Farbe so angeordnet sein, dass sie nach dem erfolgten Abmischen von der ersten Hülse 29 abgedeckt wird. Ferner kann eine zweite, andere Farbe vorgesehen sein, die nach dem Primen unter der zweiten Hülse 29 verschwindet. Beispielsweise kann nun eine dritte Farbe vorgesehen sein, die dem Verwender anzeigt, dass die Vorrichtung nun bereit ist für eine Produktausschüttung. Die äußere Hülse 29 kann z.B. mit einer reibungserhöhenden Struktur zur Verbesserung des Griffs versehen sein, wie z.B. Rippen oder Noppen oder ein reibungserhöhendes Material, wie z.B. eine Gummischicht. Gleiches gilt auch für die Flügel 57.

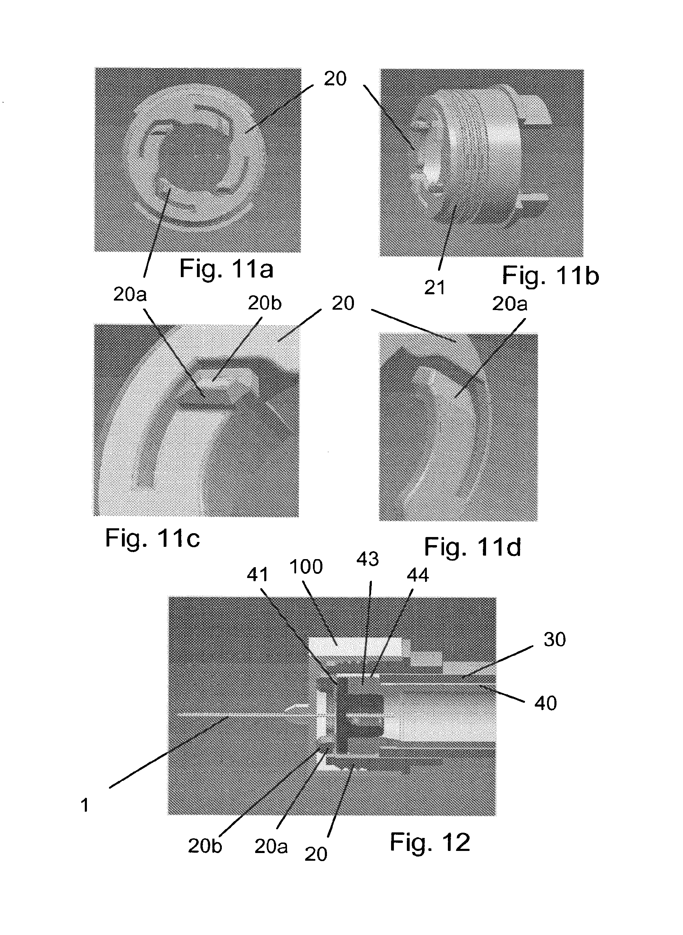

In den Figuren 11a und 11d wird ein Befestigungsglieds 20 gezeigt. In Figur 12 ist das in den Figuren 11a bis 11d gezeigte Befestigungsglied 20 an der Ampullenaufnahme 30 befestigt, wobei auf dem Befestigungsglied 20 die Nadeleinheit 100 angeordnet ist.

Das Befestigungsglied 20 kann mit einer der hierin beschriebenen Möglichkeiten mit der Ampullenaufnahme 30 verbunden werden. Das Befestigungsglied 20 weist ein Gewinde 21 auf, das beispielhaft für eine Befestigungsmöglichkeit der Nadeleinheit 100 an dem Befestigungsglied 20 ist. Alternativ können hierin beschriebene oder aus dem Stand der Technik bekannte Befestigungsmöglichkeiten für die Nadeleinheit 100 vorgesehen sein. Das Befestigungsglied 20 weist mindestens ein, in diesem Fall vier Eingriffsglieder 20a auf, die federnd jeweils an einem Arm angeordnet sind. Der Arm erstreckt sich in Umfangsrichtung, ist an einem Ende insbesondere einstückig mit dem Befestigungsglied 20 verbunden und weist an seinem anderen, nämlich freien Ende, das Eingriffsglied 20a auf. Wie am besten aus Figur 12 zu erkennen ist, wird beim Befestigen der Nadeleinheit 100 mit dem Befestigungsglied 20 das Eingriffsglied 20a gegen das distale Ende des Produktbehältnisses 30, d. h. gegen den Verschluss 41, 43, 44 gedrückt. Hierfür weist die Nadeleinheit 100 eine in proximale Richtung weisende Fläche auf, welche gegen das Eingriffsglied 20a drückt. Spätestens nach dem oder beim Anbringen der Nadeleinheit 100 an der Ampullenhalterung 20, 30 wird der Verschluss 41, 43, 44 des Produktbehältnisses 40 zwischen der Ampullenaufnahme 30 und dem Befestigungsglied 20, insbesondere dem Eingriffsglied 20a spielfrei eingefasst. Vor dem Aufsetzen der Nadeleinheit 100 kann der Verschluss 41, 43, 44 des Produktbehältnisses 40 ein leichtes axiales Spiel haben. Bereits vor dem Aufsetzen der Nadeleinheit 100 kann dieses axiale Spiel verringert oder sogar beseitigt werden, indem das Eingriffsglied 20a federnd gegen den Verschluss 41, 43, 44 drückt. Die aufgesetzte Nadeleinheit 100 hält das Eingriffsglied 20a in Anlage an die distale Stirnseite des Verschlusses 41, 43, 44.

Das Befestigungsglied 20, insbesondere das Eingriffsglied 20a kann eine Abragung 20b aufweisen, welche distal über die Stirnseite des Befestigungsglieds 20 übersteht. Hierdurch kann erreicht werden, dass das Eingriffsglied 20a noch besser gegen den Verschluss 41, 43, 44 des Produktbehältnisses 40 drücken kann. Die Abragung 20b kann ausschließlich oder bevorzugt zusätzlich die Wirkung der z. B. in den Figuren 5 und 6 gezeigten Abragungen 24 oder 25 haben, auf die hiermit Bezug genommen wird. Selbstverständlich kann die Nadeleinheit 100 die entsprechenden Elemente aufweisen, wie sie für das Zusammenwirken mit den Abragungen 24 oder 25 beschrieben werden.

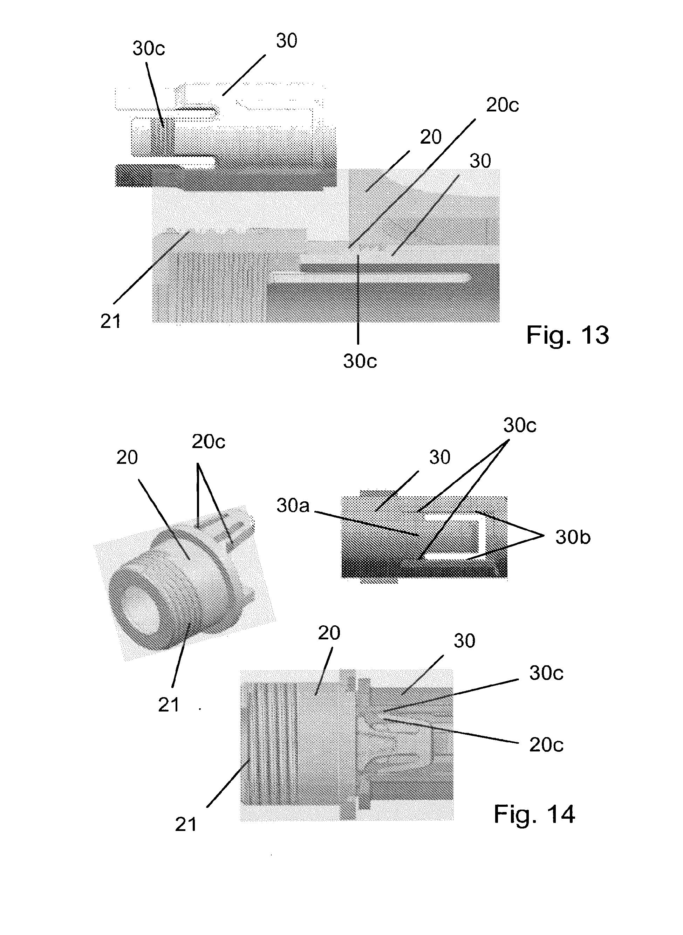

In den Figuren 13 und 14 werden Ausführungen der Injektionsvorrichtung der Erfindung gezeigt, bei denen das Befestigungsglied 20 mit einer Axialbewegung, ohne Drehbewegung auf die Ampullenaufnahme 30 gesteckt wird und mit der Ampullenaufnahme 30 verrastet. Die Rasteinrichtung ist so ausgebildet, dass das mit der Ampullenaufnahme 30 verrastete Befestigungsglied 20 in Befestigungs- oder Aufsteckrichtung verschiebbar und in entgegengesetzte Richtung nicht verschiebbar ist. Das Befestigungsglied 20 kann somit, wenn es einmal mit der Ampullenaufnahme 30 verrastet ist, nicht mehr von der Ampullenaufnahme 30 abgezogen werden, jedoch noch weiter aufgeschoben werden, wobei das Befestigungsglied 20 in diskreten Schritten in Aufsteckrichtung weiter verrastet. Mit jedem Rastschritt kann das axiale Spiel, das der Verschluss des Produktbehältnisses 40 zwischen der Ampullenaufnahme 30 und dem Befestigungsglied 20 hat, verringert werden, bis schließlich eine Rastposition erreicht ist, in der das Befestigungsglied 20 gegen den Verschluss 41, 43, 44 des Produktbehältnisses 40 drückt. Hierdurch wird der Verschluss 41, 43, 44 axial spielfrei eingefasst.

Als Rasteinrichtung weisen das Befestigungsglied 20 ein Rastmittel 20c und die Ampullenaufnahme 30 ein Rastgegenmittel 30c auf, wobei Rastmittel 20c und Rastgegenmittel 30 für die Rastverbindung ineinander greifen. In dem in Figur 13 gezeigten Beispiel ist das Rastgegenmittel 30c federnd an einem Arm angeordnet, während das Rastmittel 20c in etwa starr an dem Befestigungsglied 20 angeordnet ist. In dem in Figur 14 gezeigten Beispiel sind das Rastmittel 20c federnd an einem Arm und das Rastgegenmittel 30c im Wesentlichen starr an der Ampullenaufnahme 30 angeordnet.

Das Rastgegenmittel 30c weist mehrere in Axialrichtung hintereinander angeordnete sägezahnförmige Zähne auf, in die wenigstens ein Zahn oder mehrere Zähne des Rastmittels 20c eingreifen. Im Prinzip ist es ausreichend, wenn eines aus Rastmittel 20c und Rastgegenmittel 30c einen Zahn und das andere aus Rastmittel 20c und Rastgegenmittel 30c mehrere Zähne aufweisen, wobei für eine erhöhte Stabilität Rastmittel 20c und Rastgegenmittel 30c jeweils mehrere ineinander greifende Zähne umfassen. Die in Figur 13 gezeigte Ampullenaufnahme 30 weist an ihrem distalen Bereich Zentrierflächen und den Arm mit dem Rastgegenmittel 30c auf, wobei die Zentrierflächen das Befestigungsglied 20 radial zentrieren. Das Rastgegenglied 30c ist an der Außenseite, d. h. radial nach außen ragend an der Ampullenaufnahme 30 angeordnet.

Die in Figur 14 gezeigte Ampullenaufnahme 30 weist eine sich in Längsrichtung erstreckende Nut 30a auf, die seitlich von Nutflanken 30b eingefasst ist. Die Nutflanken 30b weisen jeweils ein Rastgegenmittel 30c auf, wobei das Rastgegenmittel 30c von der einen Nutflanke 30b in Richtung der anderen Nutflanke 30b gerichtet ist. Das Befestigungsglied 20 weist zwei jeweils an einem Arm angeordnete Rastmittel 20c auf, welche aufeinander zu und voneinander weg federn können. Die Ampullenaufnahme 30 weist eine Zentrierfläche auf, welche das Befestigungsglied 20 beim Aufstecken radial zentriert. Beim Aufstecken des Befestigungsglieds 20 geraten das Rastmittel 20c und das Rastgegenmittel 30c in einen Eingriff. Hierbei werden die Rastmittel 20c von den Nutflanken 30b aufeinander zu gedrückt, wobei durch die federnde Anordnung die Rastmittel 20c in einen Eingriff mit den Rastgegenmitteln 30c gedrückt werden. Hierdurch wird eine besonders stabile Befestigung erreicht. Grundsätzlich könnten die die Rastmittel 20c tragenden Arme sich von dem Befestigungsglied 20 in proximale Richtung erstrecken, wobei das Rastmittel 20c am proximalen Ende des Arms angeordnet ist. Eine noch bessere Anordnung wird in Figur 14 gezeigt, wobei die federnden Arme am proximalen Ende eines sich vom Befestigungsglied 20 in proximale Richtung erstreckenden Arms gebildet sind. Die federnden Arme erstrecken sich vom proximalen Ende des einen Arms in distale Richtung, wobei das Rastmittel 20c am distalen Ende des federnden Arms angeordnet ist.

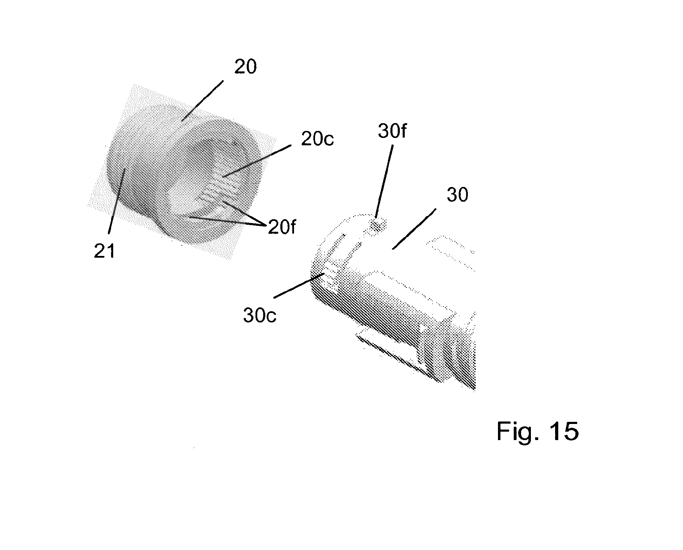

In Figur 15 wird eine Befestigungsmöglichkeit des Befestigungsglieds 20 an der Ampullenaufnahme 30 gezeigt. Im Gegensatz zu den Ausführungen aus den Figuren 13 und 14 basiert die Befestigung auf einer Steck-Dreh-Verbindung statt auf einer reinen Axialsteckverbindung. Diese Steck-Dreh-Verbindung wird im Allgemeinen auch als Bajonettverschluss 20f, 30f bezeichnet. Für den Bajonettverschluss 20f, 30f weist das Befestigungsglied 20 an seinem inneren Umfang eine nutförmige Führungsbahn 20f auf, in der ein radial nach außen ragender Nocken 30f der Ampullenaufnahme 30 einführbar ist. Durch Verdrehen des Befestigungsglieds 20 relativ zur Ampullenaufnahme 30 wird das Befestigungsglied 20 befestigt. Das Befestigungsglied 20 führt bei seiner Verdrehung bevorzugt auch eine Längsbewegung aus, so dass der Verschluss des Produktbehältnisses 40 (nicht gezeigt) zwischen Ampullenaufnahme 30 und Befestigungsglied 20 spielfrei eingefasst wird. Um ein Lösen des Befestigungsglieds 20 von der Ampullenaufnahme 30 zu verhindern, weist die Ampullenaufnahme 30 ein federnd an einem sich in Umfangsrichtung erstreckenden Arm gebildetes Rastgegenmittel 30c auf, welches in das Rastmittel 20c, das am inneren Umfang des Befestigungsglieds 20 gebildet ist, eingreifen kann. Wie bei den Ausführungen aus den Figuren 13 und 14 weisen Rastmittel 20c und Rastgegenmittel 30c sägezahnförmige Zähne auf, wobei die Zähne ebenfalls in Befestigungsrichtung hintereinander gereiht angeordnet sind. Da die Befestigungsrichtung bei der Ausführung aus Figur 15 eine Drehbewegung ist, sind die Zähne in Umfangsrichtung aneinander gereiht bzw. hintereinander gereiht angeordnet.

Statt eines Bajonettverschlusses kann für die Befestigung alternativ ein Gewinde vorgesehen sein.

## Patentansprüche

1. Ampullenhalterung (20, 30) umfassend eine Ampullenaufnahme (30), in der eine Ampulle (40), die einen Verschluss (41, 43, 44) mit einer radialen Abragung aufweist, aufnehmbar ist, und ein Befestigungsglied (20), das mit der Ampullenaufnahme (30) verbunden oder verbindbar ist, wobei die radiale Abragung des Verschlusses (41, 43, 44) zwischen Ampullenaufnahme (30) und Befestigungsglied (20) anordenbar ist, wobei eines aus Befestigungsglied (20) und Ampullenaufnahme (30) ein Rastmittel (20c) und das andere aus Befestigungsglied (20) und Ampullenaufnahme (30) ein Rastgegenmittel (30c) aufweist, wobei das Rastmittel (20c) und das Rastgegenmittel (30c) in einem Eingriff sind oder beim Befestigen des Befestigungsglieds (20) in einen Eingriff bringbar sind, **dadurch gekennzeichnet, dass** das Rastmittel (20c) und das Rastgegenmittel (30c) derart ausgebildet sind, dass sie in einer Befestigungs- oder Aufsteckrichtung, wobei das Befestigungsglied (20) mit einer Axialbewegung ohne Drehbewegung auf die Ampullenaufnahme (30) verrastbar ist, axial verschiebbar sind und in einer entgegengesetzten Richtung zur Befestigungs- oder Aufsteckrichtung axial unverschiebbar sind.

2. Ampullenhalterung nach dem vorhergehenden Anspruch, wobei das Befestigungsglied (20) ein Eingriffsglied (27; 20a) aufweist, das an das Produktbehältnis (40) angreift oder in ein Eingriff mit dem Produktbehältnis (40) bringbar ist.

3. Ampullenhalterung nach dem vorhergehenden Anspruch, wobei das Eingriffsglied (20a) federnd, insbesondere an einen Arm, der sich in Längsrichtung oder in Umfangsrichtung erstreckt, angeordnet ist.

4. Ampullenhalterung nach einem der zwei vorhergehenden Ansprüche, wobei an den an der Ampullenaufnahme (30) angeordneten Befestigungsglied (20) ein Teil, insbesondere eine Nadeleinheit (100) anbringbar ist, mit der das Eingriffsglied (20a) in einen Eingriff mit dem Produktbehältnis (40), insbesondere mit dem Verschluss (41, 43, 44) bringbar ist.

5. Ampullenhalterung nach einem der drei vorhergehenden Ansprüche, wobei das Befestigungsglied (20), insbesondere das Eingriffsglied (20a) ein Rastelement (20b) aufweist, das ein Lösen der Nadeleinheit (100) von dem Befestigungsglied (20) zumindest erschwert.

6. Ampullenhalterung nach einem der vorhergehenden Ansprüche, wobei das Befestigungsglied (20) mit einer Steckbewegung an die Ampullenaufnahme (30) anbringbar ist.

7. Ampullenhalterung nach einem der vorhergehenden Ansprüche, wobei das Befestigungsglied (20) mit der Ampullenaufnahme (30) mit einer Längsführung (30a) verbindbar oder verbunden ist.

8. Ampullenhalterung nach einem der vorhergehenden Ansprüche, wobei das Befestigungsglied (20) nur in eine Richtung, insbesondere in Befestigungsrichtung bewegbar ist und in die Gegenrichtung nicht bewegbar ist, wenn das Rastmittel (20c) und das Rastgegenmittel (30c) ineinander greifen.

9. Ampullenhalterung nach einem der vorhergehenden Ansprüche, wobei das Rastmittel (20c) mindestens einen Rastzahn und das Rastgegenmittel (30c) mindestens einen, bevorzugt mehrere Rastzähne aufweist, die so geformt sind, dass das Befestigungsglied (20) in eine Richtung bewegbar und in die andere Richtung nicht bewegbar ist, wobei die Rastzähne bevorzugt sägezahnförmig geformt sind.

10. Ampullenhalterung nach dem vorhergehenden Anspruch, wobei die Rastzähne in Befestigungsrichtung, insbesondere in Umfangsrichtung oder in Längsrichtung aufeinanderfolgend angeordnet, insbesondere aneinandergereiht sind.

11. Ampullenhalterung nach einem der vorhergehenden Ansprüche, wobei wenigstens eines aus Rastmittel (20c) und Rastgegenmittel (30c) federnd angeordnet ist.

12. Ampullenhalterung nach einem der vorhergehenden Ansprüche, wobei die Ampullenaufnahme (30) eine Nut (30a) aufweist, wobei eine oder beide zueinander weisenden Nutflanken (30b) jeweils ein Rastgegenmittel (30c) aufweisen und wobei das Befestigungsglied (20) mindestens ein Rastmittel (20c) aufweist, wobei das mindestens eine Rastmittel (20c) federnd gegen die ihr zugeordnete Nutflanke, insbesondere in das Rastgegenmittel (20c) gedrückt werden.

13. Ampullenhalterung nach einem der vorhergehenden Ansprüche, wobei das Rastgegenmittel (30c) mehrere in Axialrichtung hintereinander angeordnete sägezahnförmige Zähne aufweist, in die wenigstens ein Zahn oder mehrere Zähne des Rastmittels (20c) eingreifen oder in Eingriff bringbar sind, wobei die Zähne des Rastmittels (20c) und des Rastgegenmittels (30c) derart ausgebildet sind, dass sie in einer Befestigungs- oder Aufsteckrichtung relativ zueinander axial verschiebbar sind und in einer entgegengesetzten Richtung zur Befestigungs- oder Aufsteckrichtung relativ zueinander axial unverschiebbar sind.

14. Ampullenhalterung nach einem der vorhergehenden Ansprüche, wobei die Ampullenhalterung wenigstens eines der folgenden Merkmale aufweist:
- die Ampulle (40) ist stirnseitig mit ihrem zylindrischen Gehäuse (48) voran in die Ampullenaufnahme (30) einführbar,
- zumindest eines aus Ampullenaufnahme (30) und Befestigungsglied (20) weisen wenigstens eine gegen den Verschluss gerichtete Abragung (35; 27) auf,
- wenigstens eine Abragung (35) ist an der Stirnseite oder an einer radialen Abragung (28) des Befestigungsglieds (20) gebildet,
- die Ampullenaufnahme weist eine sich aus Richtung der Stirnseite her entlang der Ampullenaufnahme (30) erstreckende Aussparung für einen Bypass (47) einer Zweikammerampulle (40) auf.

15. Kombination aus der Ampullenhalterung (20, 30) nach einem der vorhergehenden Ansprüche und einer Ampulle (40), wobei die Ampulle (40) ein zylindrisches Gehäuse (48) mit einem Aussendurchmesser und einem Verschluss (41, 43, 44) umfasst, der eine Stirnseite des Gehäuses (40) verschliesst, wobei der Verschluss (41, 43, 44) radial über den Aussendurchmesser ragt, wobei die Ampulle (40) insbesondere wenigstens eines der folgenden Merkmale aufweist:
- die Ampulle (40) ist eine Zweikammerampulle
- der Verschluss (41, 43, 44) ist mit einem Stechmittel (2) einer Nadeleinheit (100) durchstechbar,
- der Verschluss (41, 43, 44) weist ein Septum (41) auf, das zumindest im Bereich der radialen Abragung von einem ringförmigen Metallteil eingefasst ist.

## Claims

1. Ampoule holder (20, 30) comprising an ampoule receptacle (30), in which an ampoule (40) having a seal (41,43,44) with a radial projection is accommodated, and a fastening member (20) which is connected or connectable to the ampoule receptacle (30), wherein the radial projection of the seal (41, 43, 44) can be arranged between ampoule receptacle (30) and fastening member (20), wherein a latching means (20c) is provided on the fastening member (20) or the ampoule receptacle (30) and the other has a latching counter means (30c), wherein the latching means (20c) and the latching counter means (30c) mesh together or, when securing the fastening member (20), they can mesh together, **characterised in that** the latching means (20c) and the latching counter means (30c) are formed such that they are movable axially in a fastening or insertion direction, wherein the fastening member (20) is latchable to the ampoule receptacle (30) without a rotational movement, and cannot be moved axially in a direction opposite the fastening or insertion direction.

2. Ampoule holder according to one of the preceding claims, wherein the fastening member (20) has an engagement member (27; 20a) which grips on to the product container (40) or can be meshed with the product container (40).

3. Ampoule holder according to one of the preceding claims, wherein the engagement member (20a) is arranged to be resilient, in particular on an arm which extends in a longitudinal direction or in a circumferential direction.

4. Ampoule holder according to one of the two preceding claims, wherein a part, in particular a needle unit (100) can be fitted to the fastening member (20) arranged on the ampoule receptacle (30), wherein the engagement member (20a) can be connected to the product container (40), in particular with the seal (41, 43, 44), by means of said needle unit.

5. Ampoule holder according to one of the three preceding claims, wherein the fastening member (20), in particular the engagement member (20a), has a latching means (20b) which makes it difficult to release the needle unit (100) from the fastening member (20).

6. Ampoule holder according to one of the preceding claims, wherein the fastening member (20) can be fitted to the ampoule receptacle (30) with an insertion movement.

7. Ampoule holder according to one of the preceding claims, wherein the fastening member (20) can be connected or is connected to the ampoule receptacle (30) with a longitudinal guide (30a).

8. Ampoule holder according to one of the preceding claims, wherein the fastening member (20) can be moved in one direction only, in particular in a fastening direction and cannot be moved in the opposite direction if the latching means (20c) and the latching counter means (30c) mesh with each other.

9. Ampoule holder according to one of the preceding claims, wherein the latching means (20c) has at least one latching tooth and the latching counter means (30c) has at least one tooth, preferably a multiplicity of latching teeth, which are shaped such that the fastening member (20) can be moved in one direction and cannot be moved in the other direction, wherein the latching teeth are preferably saw-toothed in shape.

10. Ampoule holder according to the preceding claim, wherein the latching teeth are arranged consecutively, in particular in rows, in the fastening direction, in particular in a circumferential direction or in a longitudinal direction

11. Ampoule holder according to one of the preceding claims, wherein at least one out of the latching means (20c) or the latching counter means (30c) is arranged to be resilient.

12. Ampoule holder according to one of the preceding claims, wherein the ampoule receptacle (30) has a groove (30a) wherein one flank or both groove flanks facing each other each have a latching counter means (30c) and wherein the fastening member (20) has at least one latching means (20c), wherein the at least one latching means (20c) be pressed resiliently against the groove flank associated with it, in particular into the latching counter means (20c).

13. Ampoule holder according to one of the preceding claims, wherein the latching counter means (30c) has a multiplicity of saw-tooth shaped teeth arranged one after the other in an axial direction, in which at least one tooth or a multiplicity of teeth of the latching means (20c) mesh or can be meshed, wherein the teeth of the latching means (20c) and of the latching counter means (30c) are formed such that they can be moved axially relative to each other in a fastening or insertion direction and cannot be moved axially relative to each other in a direction opposite the fastening or insertion direction.

14. Ampoule holder according to one of the preceding claims, wherein the ampoule holder has at least one of the following features:
- the ampoule (40) can be inserted into the ampoule receptacle (30) at the front end with its cylindrical housing (48) leading.
- at least one out of the ampoule receptacle (30) and the fastening member (20) has at least one projection (35, 27) pointing towards the seal,
- at least one projection (35) is formed on the front face or on a radial projection (28) of the fastening member (20)
- the ampoule receptacle has a recess for a bypass (47) of a two-chamber ampoule (40) extending along the ampoule receptacle (30).

15. Combination of the ampoule holder (20, 30) according to one of the preceding claims and an ampoule (40), wherein the ampoule (40) comprises a cylindrical housing (48) with an outer diameter and a seal (41, 43, 44) which seals one end face of the housing (40), wherein the seal (41, 43, 44) projects radially beyond the outer diameter, wherein the ampoule (40) has in particular at least one of the following features:
- the ampoule (40) is a two-chamber ampoule,
- the seal (41, 43, 44) can be pierced with a piercing means (2) of a needle unit (100)
- the seal (41, 43, 44) has a septum (41) which is surrounded at least in the region of the radial projection by a ring-shaped metal part.

## Revendications

1. Support pour ampoule (20, 30) comprenant un logement d'ampoule (30), dans lequel une ampoule (40) qui présente une fermeture (41, 43, 44) avec une saillie radiale peut être reçue, et un organe de fixation (20), qui est relié ou peut être relié avec le logement d'ampoule (30), étant entendu que la saillie radiale de la fermeture (41, 43, 44) peut être agencée entre le logement d'ampoule (30) et l'organe de fixation (20), étant entendu que l'un parmi l'organe de fixation (20) et le logement d'ampoule (30) présente un moyen d'encliquetage (20c) et l'autre parmi l'organe de fixation (20) et le logement d'ampoule (30) présente un moyen d'encliquetage opposé (30c), étant entendu que le moyen d'encliquetage (20c) et le moyen d'encliquetage opposé (30c) sont en prise ou peuvent être mis en prise lors de la fixation de l'organe de fixation (20), **caractérisé en ce que** le moyen d'encliquetage (20c) et le moyen d'encliquetage opposé (30c) sont réalisés de telle sorte qu'ils peuvent être déplacés axialement dans une direction de fixation ou de montage, ce par quoi l'organe de fixation (20) peut être encliqueté sur le logement d'ampoule (30) avec un mouvement axial sans mouvement rotatif, et qu'ils ne peuvent pas être déplacés axialement dans une direction opposée à la direction de fixation ou de montage.

2. Support pour ampoule selon la revendication précédente, dans lequel l'organe de fixation (20) présente un organe de prise (27 ; 20a), qui s'engage avec le contenant de produit (40) ou peut être mis en prise avec le contenant de produit (40).

3. Support pour ampoule selon la revendication précédente, dans lequel l'organe de prise (20a) est agencé de façon élastique, en particulier sur un bras qui s'étend dans la direction longitudinale ou dans la direction circonférentielle.

4. Support pour ampoule selon l'une des deux revendications précédentes, dans lequel sur l'organe de fixation (20) agencé sur le logement d'ampoule (30), on peut installer une pièce, en particulier une unité d'aiguille (100), avec laquelle l'organe de prise (20a) peut être mis en prise avec le contenant de produit (40), en particulier avec la fermeture (41, 43, 44).

5. Support pour ampoule selon l'une des trois revendications précédentes, dans lequel l'organe de fixation (20), en particulier l'organe de prise (20a), présente un élément d'encliquetage (20b) qui rend au moins difficile une séparation de l'unité d'aiguille (100) de l'organe de fixation (20).

6. Support pour ampoule selon l'une des revendications précédentes, dans lequel l'organe de fixation (20) peut être installé sur le logement d'ampoule (30) avec un mouvement d'enfichage.

7. Support pour ampoule selon l'une des revendications précédentes, dans lequel l'organe de fixation (20) peut être relié ou est relié avec le logement d'ampoule (30) avec un guide longitudinal (30a).

8. Support pour ampoule selon l'une des revendications précédentes, dans lequel l'organe de fixation (20) peut seulement être déplacé dans une direction, en particulier dans la direction de fixation, et ne peut pas être déplacé dans la direction opposée lorsque le moyen d'encliquetage (20c) et le moyen d'encliquetage opposé (30c) sont engagés l'un dans l'autre.

9. Support pour ampoule selon l'une des revendications précédentes, dans lequel le moyen d'encliquetage (20c) présente au moins une dent d'encliquetage et le moyen d'encliquetage opposé (30c) présente au moins une, et de préférence plusieurs dents d'encliquetage, qui sont formées de telle sorte que l'organe de fixation (20) peut être déplacé dans une direction et ne peut pas être déplacé dans l'autre direction, étant entendu que les dents d'encliquetage sont de préférence réalisées en forme de dents de scie.

10. Support pour ampoule selon la revendication précédente, dans lequel les dents d'encliquetage sont agencées l'une à la suite de l'autre, en particulier alignées l'une à côté de l'autre, dans la direction de fixation, en particulier dans la direction circonférentielle ou dans la direction longitudinale.

11. Support pour ampoule selon l'une des revendications précédentes, dans lequel au moins l'un des moyens parmi le moyen d'encliquetage (20c) et le moyen d'encliquetage opposé (30c) est agencé de façon élastique.

12. Support pour ampoule selon l'une des revendications précédentes, dans lequel le logement d'ampoule (30) présente une rainure (30a), étant entendu qu'un flanc ou les deux flancs de rainure (30b) dirigés l'un vers l'autre présentent respectivement un moyen d'encliquetage opposé (30c) et étant entendu que l'organe de fixation (20) présente au moins un moyen d'encliquetage (20c), étant entendu que l'au moins un moyen d'encliquetage (20c) est comprimé de façon élastique contre le flanc de rainure qui lui correspond, en particulier dans le moyen d'encliquetage opposé (20c).

13. Support pour ampoule selon l'une des revendications précédentes, dans lequel le moyen d'encliquetage opposé (30c) présente plusieurs dents en forme de dents de scie agencées l'une derrière l'autre dans la direction axiale, dans lesquelles au moins une dent ou plusieurs dents du moyen d'encliquetage (20c) s'engagent ou peuvent être mises en prise, étant entendu que les dents du moyen d'encliquetage (20c) et du moyen d'encliquetage opposé (30c) sont réalisées de telle sorte qu'elles peuvent être déplacées axialement les unes par rapport aux autres dans une direction de fixation ou de montage et qu'elles ne peuvent pas être déplacées axialement les unes par rapport aux autres dans une direction opposée à la direction de fixation ou de montage.

14. Support pour ampoule selon l'une des revendications précédentes, dans lequel le support pour ampoule présente au moins l'une des caractéristiques suivantes :
- l'ampoule (40) peut être introduite dans le logement d'ampoule (30) du côté frontal avec son boîtier cylindrique (48) en avant ;
- au moins un élément parmi le logement d'ampoule (30) et l'organe de fixation (20) présente au moins une saillie dirigée contre la fermeture (35 ; 27) ;
- au moins une saillie (35) est formée sur le côté frontal ou sur une saillie radiale (28) de l'organe de fixation (20) ;
- le logement d'ampoule présente un évidement s'étendant depuis la direction du côté frontal le long du logement d'ampoule (30) pour une dérivation (47) d'une ampoule à deux compartiments (40).

15. Combinaison du support pour ampoule (20, 30) selon l'une des revendications précédentes et d'une ampoule (40), étant entendu que l'ampoule (40) comprend un boîtier cylindrique (48) avec un diamètre extérieur et une fermeture (41, 43, 44) qui ferme un côté frontal du boîtier (40), étant entendu que la fermeture (41, 43, 44) fait saillie radialement au-delà du diamètre extérieur, étant entendu que l'ampoule (40) présente en particulier au moins l'une des caractéristiques suivantes :
- l'ampoule (40) est une ampoule à deux compartiments ;
- la fermeture (41, 43, 44) peut être percée avec un moyen de perçage (2) d'une unité d'aiguille (100) ;
- la fermeture (41, 43, 44) présente un diaphragme (41) qui est entouré par une pièce métallique de forme annulaire au moins dans la zone de la saillie radiale.
